# EUROPEAN PATENT SPECIFICATION

(11) **EP 3 923 843 B1**
(45) Date of publication and mention of the grant of the patent: **09.04.2025**
(21) Application number: 20704033.8
(22) Date of filing: 13.02.2020
(51) Int. Cl.: A61B 18/14

(54) **AN ABLATION PROBE**
ABLATIONSSONDE
SONDE D'ABLATION

(30) Priority: 13.02.2019 EP 19157020
(43) Date of publication of application: 22.12.2021
(73) Proprietor: University of Galway, H91 TK33 Galway (IE)
(72) Inventor: EATON-EVANS, Jimmy, Galway University Road Galway (IE); RUVIO, Giuseppe, Galway University Road Galway (IE); BOUCHIER-HAYES, Jonathan, Galway University Road Galway (IE); O'HALLORAN, Martin, Galway University Road Galway (IE); BRUZZI, Mark, Galway University Road Galway (IE)
(74) Representative: Barker Brettell LLP
(86) International application number: PCT/EP2020/053819
(87) International publication number: WO 2020/165375

(56) References cited:
- EP-A1- 3 381 393
- EP-A1- 3 381 393
- GB-A- 2 547 941
- GB-A- 2 547 941
- US-A1- 2004 133 254
- US-A1- 2004 133 254
- US-A1- 2007 203 551
- US-A1- 2007 203 551
- US-A1- 2011 213 352
- US-A1- 2011 213 352
- US-A1- 2011 319 880
- US-A1- 2011 319 880
- US-A1- 2013 178 841
- US-A1- 2013 178 841
- US-A1- 2013 178 842

## Description

This application relates to an ablation probe. In particular, this application relates to an ablation probe that may be used to generate heat within tissue to destroy tissue growths.

Thermal ablation can be used to destroy tissue growths within the body which can be malignant. Current ablation systems use applicators that deliver Radio Frequency (RF) energy (or microwave energy) to the tissue surrounding the applicator tip. This causes localised heating and destruction of the malignant cells. These applicators may be designed for percutaneous delivery and are therefore relatively short in length and large in diameter. However, many disease locations cannot be safely or easily accessed percutaneously. For example, the location of the pancreas behind the liver makes it difficult to access percutaneously. Similarly, access to the lung through the chest wall can cause a pneumothorax. Large diameter applicators may also cause undesired tissue damage during insertion. This limits the range of indications where thermal ablation therapy can be successfully delivered using existing percutaneous applicators.

Various sites within the human body can be accessed by navigating through a natural orifice. For example the periphery of the lung can be accessed using lung navigation systems, or similar devices such as an endoscope, that guide a working channel through the airway network to a target. This enables therapies to be delivered through the device working channel to diagnose and treat disease. Microwave ablation can be delivered via these systems. However, a long and flexible ablation catheter is required that is capable of delivering sufficient power to its radiating tip. Known microwave systems use coaxial cable to deliver power, with larger diameter cables used to generate fewer electrical losses than smaller gauge cables. However, small diameter cables improve flexibility, reduce insertion profile and require less force to straighten if plastically deformed during delivery. It is not practical to run a small cable (e.g. diameter < 0.7 mm) over the length necessary to reach many target sites (e.g. >1 m for lung) because the electrical losses would be too great, and may result in excessive heating effects and insufficient power delivery (resulting in excessively long treatment times).

**In** the applicant's previous European application No. EP17164403.2 (EP3381393A1) filed on 31 March 2017, a microwave ablation probe having a feeding cable arranged to supply electromagnetic energy to an applicator was disclosed. The feeding cable comprises a proximal portion and a distal portion having different cross section sizes to each other. A connector is also provided to mechanically and electrically splice the distal portion of the feeding cable to the proximal portion. EP17164403.2 also disclosed the use of a deformable member which provides a coolant path through which coolant is able to flow.

The applicant is also aware of the following: US2004133254A1 discloses inflatable balloon catheter structural designs and methods for treating diseased tissue of a patient. US2013178841A1 discloses ablation systems, probes, and methods for reducing radiation from an ablation probe into the environment. GB2547941A discloses an electrosurgical instrument. US2011213352A1 discloses a tunable microwave ablation probe. US2011319880A1 discloses a microwave ground plane antenna probe. US2007203551A1 discloses a radiation applicator and method of radiating tissue.

Improvements to known ablation probes such as this are desired to allow efficient use with delivery devices such as endoscopes or Electromagnetic Navigation Bronchoscopy (ENB) Systems. A suitable level of flexibility must be provided so that a tortuous route through anatomy can be followed (e.g. bend radius of less than 10mm). The ablation probe must also be compact in size so that it can be used with a narrow working channel (e.g. 2 mm diameter). Reducing the size of the ablation probe can lead to problems in maintaining mechanical strength so that the device does not fail during delivery or therapy. A secure connection between components is therefore desired, which can be difficult to achieve where components are formed from different, incompatible, materials. Moreover, a simple geometry is desired to help aid manufacture and allow volume production and reliability. The overall assembly must be flexible enough for delivery through a tortuous path to reach the disease location.

The present application provides an ablation probe as defined in claim 1.

The coupling body acts as a single component with which the applicator and tubular member are secured together. By encapsulating the applicator within the coupling body a secure mechanical connection to the applicator can be made with less reliance on adhesives or machining the applicator (which may be formed from a dielectric) into a complex shape. The material of the coupling body may be different from that of the applicator and so more compatible for bonding to other parts of the ablation probe during assembly.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

The coupling body is formed from nylon or glass reinforced nylon. The tubular member may optionally be formed at least partly from a polymeric material such as Nylon, Pebax or PEEK. This may aid thermal welding of the coupling body and tubular member.

The deformable member is formed at least partly from nylon. This may aid thermal welding of the coupling body and deformable member.

Optionally, the applicator may comprise an antenna (or applicator) body and an antenna conductor, wherein the antenna body comprises an outer surface having a channel in which the antenna conductor is received. The antenna conductor may be formed from an elongate wire component, with the channel following a path around and/or along the outer surface having a shape corresponding (e.g. the same as) to the shape of the path followed by the antenna conductor. The channel is therefore adapted to maintain the shape of the antenna conductor. Optionally, the channel may extend along a helical path around a central axis of the antenna body.

Optionally, the applicator may comprise an applicator body and an antenna conductor. The antenna conductor may be arranged on an outer surface of the applicator body. A potting material or adhesive may be provided to secure the antenna conductor in position. This may avoid the machining of channels in the applicator body.

The antenna conductor may extend along a helical path around the outer surface of the applicator body.

Optionally, the feeding cable may comprise an inner conductor, an outer conductor and a dielectric material between them, and wherein the antenna conductor is formed from part of the inner conductor of the feeding cable.

Optionally part of the length of the antenna conductor extends in a distal direction from a distal end of the outer conductor and within an axial through hole formed in the antenna body.

Optionally, a potting compound or adhesive is provided between the applicator and the coupling body.

Optionally, the coupling body is insert moulded around the applicator.

Optionally, the coupling interface at which the coupling body is coupled to the tubular member may comprise an overlapping portion of the coupling body that extends within or around the tubular member so that they overlap one another.

Optionally, the overlapping portion forms a reduced thickness portion of the coupling body overlapping the tubular member. This may help to enhance flexibility or strain relief at this joint.

Optionally, a welded or reflowed joint is formed between the coupling body and the tubular member. This may provide a strong and secure coupling between the components.

Optionally, the pointed distal tip of the coupling body may be provided by a separate tip member coupled to the distal end of the coupling body. The tip member may be coupled via an interlocking profile (e.g. a socket) provided on the distal end of the coupling body. The tip member may be formed from a metal, metal alloy or ceramic material.

Optionally, the ablation probe further comprises a sheath member movable between a first position in which it surrounds the pointed tip of the coupling body and a second position in which the pointed tip is uncovered.

Optionally, the sheath member may extend part way along the length of the ablation probe between the distal and proximal ends of the ablation probe, and wherein the ablation probe comprises one or more control lines connected to the sheath member, the control lines extending along the length of the ablation probe between the sheath member and a position at or near the proximal end of the ablation probe.

Optionally the one or more control lines may be connected at or near a distal end of the sheath member.

Optionally, the sheath member is formed from a tube having a reinforcing ring at its distal end, wherein the one or more control lines are connected to the reinforcing ring.

Optionally, the ablation probe is slidably coupled to a handle provided at or near its proximal end, and wherein the one or more control lines are connected between the handle and the sheath member, and the control lines are arranged to restrict the range of movement of the sheath in a proximal direction away from the handle.

Optionally, the ablation probe comprises an outer catheter tube in which part of the coolant flow path is contained, and wherein the one or more control lines extend within a channel or lumen formed in the outer catheter tube.

Optionally, the sheath member forms a friction fit with the body of the ablation probe around which it extends and/or comprises a biasing member arranged to bias the sheath member in a distal direction.

Optionally, the sheath member may comprise a covering member arranged to cover the pointed tip when the sheath member is in the first position, the covering member being pierced by the pointed tip when the sheath member is moved from the first position to the second position to expose the pointed tip. The covering member may be a membrane extending across the distal end of the sheath member.

Optionally, the ablation probe may further comprise one or more spacer members, each arranged to space apart the interior walls of a channel or channels forming the coolant flow path. The channel or channels forming the coolant flow path may be generally annular in cross section. At least part of each of the one or more spacer members may be generally helical in shape. The helical spacer members may form a corresponding helical coolant flow passage therethrough. The helical shape of the spacer members may maintain flexibility and coolant flow rate, while reducing the risk of the coolant channel being deformed when the ablation probe is bent.

Optionally, the coolant flow path comprises a first coolant flow path and a second coolant flow path. The first and second coolant flow paths may be fluidly connected by one or more connecting holes extending through respective walls of the channels forming the first and second coolant flow paths. One of the one or more spacer members may be located at a position overlapping the one or more connecting holes. This may prevent deformation of the coolant channels at a position where the holes would make it otherwise more likely.

Optionally, the feeding cable comprises a portion of its length having a relatively greater flexibility compared to an adjacent proximal portion of its length, the portion of greater flexibility being located at or near a point of connection between the feeding cable and the applicator (i.e. in a distal region of the feeding cable). Optionally the portion of greater flexibility is formed by a weakened portion of an outer conductor forming the feeding cable. The greater flexibility of the feeding cable helps to avoid resistance to changes in shape of the ablation probe when inserted through tortuous anatomy, and also helps ensure the ablation probe returns back to its original shape when bent. This may specifically be advantageous in the portion of the feeding cable that is inserted into tissue during use.

The portion of greater flexibility may be formed by a weakened portion of an outer conductor forming the feeding cable. The portion of greater flexibility may comprise an indentation or slot formed in the outer conductor of the feeding cable. The indentation or slot may extend along a helical path along the length of the feeding cable.

The portion of greater flexibility may have a varying flexibility along the length of the feeding cable. The flexibility may vary smoothly at the boundary with the adjacent proximal part of the feeding cable.

Optionally, the feeding cable comprises a strain relief portion extending along the length of the feeding cable from a position at or near to the point of connection between the feeding cable and applicator. The strain relief portion may be formed by a length of the feeding cable that has a decreasing flexibility when moving along the length of the feeding cable in the distal direction towards to the applicator. This may provide a smoother transition between parts of the ablation probe having different levels of flexibility. The strain relief portion may be formed by a varying degree of weakening or reinforcement along the associated length of the feeding cable. In some embodiments, both the strain relief portion and the portion of greater flexibility are provided. In such an embodiment, the strain relief portion is located between the applicator and the greater flexibility portion.

Optionally, the applicator may extend between a distal end and a proximal end. The ablation probe may further comprise a varying flexibility portion extending along its length from a position adjacent (or level with) the distal or proximal end of the applicator. The varying flexibility portion may comprise a region of the ablation probe that has a minimum degree of flexibility closest to the applicator and which increases in flexibility in a direction extending along the length of the ablation probe away from the applicator. This may provide a smoother variation of flexibility in the region of the ablation probe where the applicator is located. The varying flexibility portion may be provided as part of the coupling body or the tubular member.

Optionally, the varying flexibility portion may be formed by a region of the ablation probe comprising a reinforcing material. The reinforcing material may be arranged to vary in shape and/or distribution to vary the flexibility of the varying flexibility portion.

Optionally, the reinforcing material comprises a helical fibre or wire having a pitch, the pitch decreasing along the length of the ablation probe in a direction towards the applicator (i.e. the axial spacing between the turns of the helix decreases closer to the applicator). This may provide the desired change in flexibility toward the boundary of the region of the ablation probe where the applicator is located.

**In** a third aspect, the present application provides an ablation probe, comprising any one or more of:
an applicator arranged to apply radiation to heat surrounding tissue;
a feeding cable arranged to supply electromagnetic energy to the applicator;
a pointed distal tip adapted for piercing tissue;
a sheath member movable between a first position in which it surrounds the pointed tip and a second position in which the pointed tip is uncovered, the sheath member extending part way along the length of the ablation probe the distal and proximal ends of the ablation probe; and
one or more control lines connected to the sheath member, the control lines extending along the length of the ablation probe between the sheath member and a position at or near the proximal end of the ablation probe.

In a fourth aspect, the present application provides a ablation probe assembly, comprising:
a handle
an ablation probe moveably coupled relative to the handle between an extended and retracted position, the ablation probe comprising:
   an applicator arranged to apply radiation to heat surrounding tissue;
   a feeding cable arranged to supply electromagnetic energy to the applicator;
   a pointed distal tip adapted for piercing tissue;
   a sheath member movable between a first position in which it surrounds the pointed tip and a second position in which the pointed tip is uncovered, sheath member extending part way along the length of the ablation probe the distal and proximal ends of the ablation probe; and
   one or more control lines connected to the sheath member, the control lines extending along the length of the ablation probe between the sheath member and the handle, whereby movement of the ablation probe relative to the handle from the retracted to the extended position causes movement of the sheath member from the first position to the second position.

By coupling the sheath member via one or more control lines its position can be controlled without its length running along the full length of the ablation probe back to the proximal end. This saves space taken up by the device in the working channel of a delivery device with which it is used.

The following statements apply to the third and fourth aspects:
Optionally the one or more control lines may be connected at or near a distal end of the sheath member.

Optionally, the sheath member is formed from a tube having a reinforcing ring at its distal end, wherein the one or more control lines are connected to the reinforcing ring.

Optionally, the ablation probe is slidably coupled to a handle provided at or near its proximal end, and wherein the one or more control lines are connected between the handle and the sheath member, and the control lines are arranged to restrict the range of movement of the sheath in a proximal direction away from the handle.

Optionally, the ablation probe comprises an outer catheter tube in which part of the coolant flow path is contained, and wherein the one or more control lines extend within a channel or lumen formed in the outer catheter tube.

Optionally, the sheath member forms a friction fit with the body of the ablation probe around which it extends and/or comprises a biasing member arranged to bias the sheath member in a distal direction.

Optionally, the sheath member may comprise a covering member arranged to cover the pointed tip when the sheath member is in the first position, the covering member being pierced by the pointed tip when the sheath member is moved from the first position to the second position to expose the pointed tip. The covering member may be a membrane extending across the distal end of the sheath member.

**In** a fifth aspect, there is provided an ablation probe comprising:
an applicator arranged to apply radiation to heat surrounding tissue;
a feeding cable arranged to supply electromagnetic energy to the applicator;
a coolant flow path forming a coolant supply circuit, the coolant flow path defined by the interior walls of one or more channels;
one or more spacer members each arranged to space apart the interior walls of the one or more channels;
wherein the one or more channels forming the coolant flow path are generally annular in cross section, and at least part of each of the one or more spacer members is generally helical in shape.

The spacer members of the fifth aspect may be as defined elsewhere herein. The spacer members may be provided in addition to, and are separate from, a choke electrically coupled to the applicator and which extends around the feeding cable. The spacer members may be electrically insulating so that they do not provide an electric coupling between the channel walls which they are arranged to space apart, or are provided at a distance proximal from the applicator so as not to operate as a choke.

Any of the features disclosed in the statements above (or elsewhere herein) in connection with one aspect may be provided in combination with the any another aspect.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness, it is also stated that the term "comprising" does not exclude other elements or steps, the term "a" or "an" does not exclude a plurality.

Embodiments of the invention will now be described, by way of example only, with reference to the accompanying drawings, in which:
Figure 1 shows a side view of an ablation probe according to an embodiment;
Figure 2 shows an exploded view of part of the ablation probe shown in Figure 1;
Figure 3 shows a cross section view of part of the ablation probe shown in Figure 1;
Figure 4 shows another cross section view of part of the ablation probe shown in Figure 1;
Figure 5 shows a close up view of the applicator of the ablation probe shown in Figure 1;
Figure 6 shows a close up cross sectional view of the region around the applicator of an ablation probe according to another embodiment;
Figure 7 shows a close up cross sectional view of the region around the applicator of the ablation probe according to another embodiment;
Figures 8 and 9 show close up side views of embodiments of the ablation probe having a varying flexibility portion;
Figure 10a shows a close up cross sectional view of a distal end of the feeding cable of another embodiment of the ablation probe;
Figures 10b and 10c show close up side and cross section views respectively of the feeding cable of the embodiment shown in Figure 10a;
Figure 10d shows another close up cross sectional view of a distal end of the feeding cable according to another embodiment of the ablation probe;
Figure 11 shows a close up cross section view of the boundary between a needle portion and a catheter portion of the ablation probe shown in Figure 1;
Figure 12a shows a cross section view of an embodiment of the ablation probe having spacer members;
Figure 12b shows an exploded view of another embodiment of the ablation probe having spacer members;
Figures 13 and 14 show close up cross sectional views of spacer members according to other embodiments;
Figure 15 shows a close up cross sectional view of an embodiment having a spacer member located with a choke;
Figures 16a and 16b show side views of an ablation probe having a sheath member according to another embodiment;
Figure 17 shows a close up cross section view of the sheath member of the ablation probe shown in Figures 16a and 16b;
Figures 18a and 18b show side views of the ablation probe shown in Figure 16a being moved relative to a handle to which the ablation probe is coupled; and
**Figure 19** shows an ablation probe having a sheath according to another embodiment.

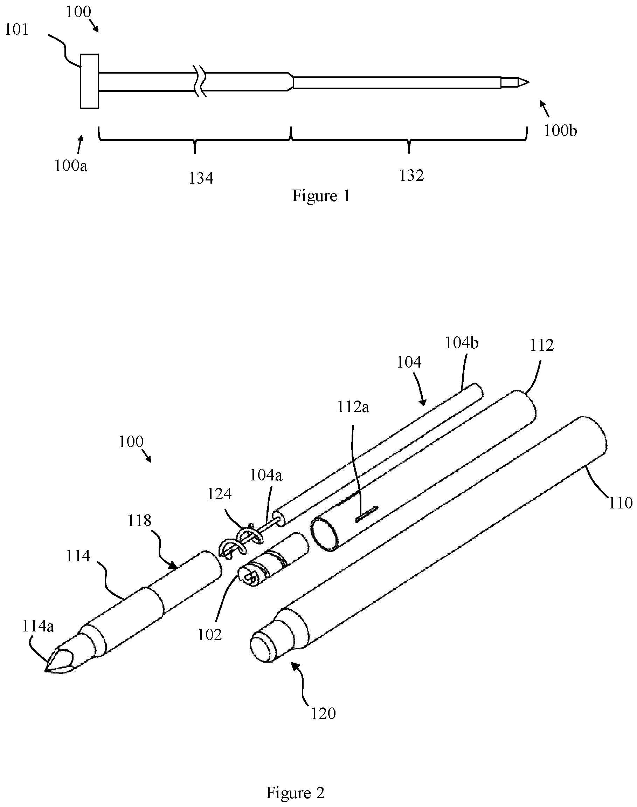

An ablation probe 100 according to an embodiment is shown schematically in Figure 1. The ablation probe 100 of the present disclosure may be suitable for insertion into the body to reach a desired treatment site, such as a malignant tissue growth. In order to reach a desired treatment site, the ablation probe may be suitable for insertion through the working channel of an internal anatomy access device. By internal anatomy access device we mean any device which may be placed within the anatomy of a patient, the device having a working channel for insertion of instruments to a desired location within the body. The internal anatomy device may be an intraluminal delivery device arranged to be delivered along an anatomical lumen of the patient (e.g. the trachea and the pathways of the bronchi in the lungs or the oesophagus). The ablation probe 100 may, for example, be used endoscopically or using an ENB system in order to reach a variety of disease locations within the body. The ablation probe may therefore have an overall flexibility such that it can be inserted through the working channel of the endoscope. In other embodiments, the ablation probe may be used with other types of intraluminal delivery device such as specific types of endoscope (e.g. a bronchoscope) or a navigation system such as a lung navigation system (e.g. an ENB system). In other examples, the ablation probe 100 may also be used percutaneously, or using any other suitable technique, e.g. inserted through an existing aperture of the body. For percutaneous use, the ablation probe may be generally rigid so that it can be inserted.

The ablation probe extends between a proximal end 100a and a distal end 100b. The terms "distal" and "proximal" are taken relative to the user operating the ablation probe and the treatment site when the ablation probe is positioned for use - the distal end 100b of the ablation probe 100 is that closest to the treatment site and the proximal end 100a is that closest to the user. A handle 101 is provided at the proximal end of the ablation probe 100 so that it can be manipulated and positioned by the user. The distal end 100b may be fed through the working channel of an endoscope or similar device to reach a target ablation site.

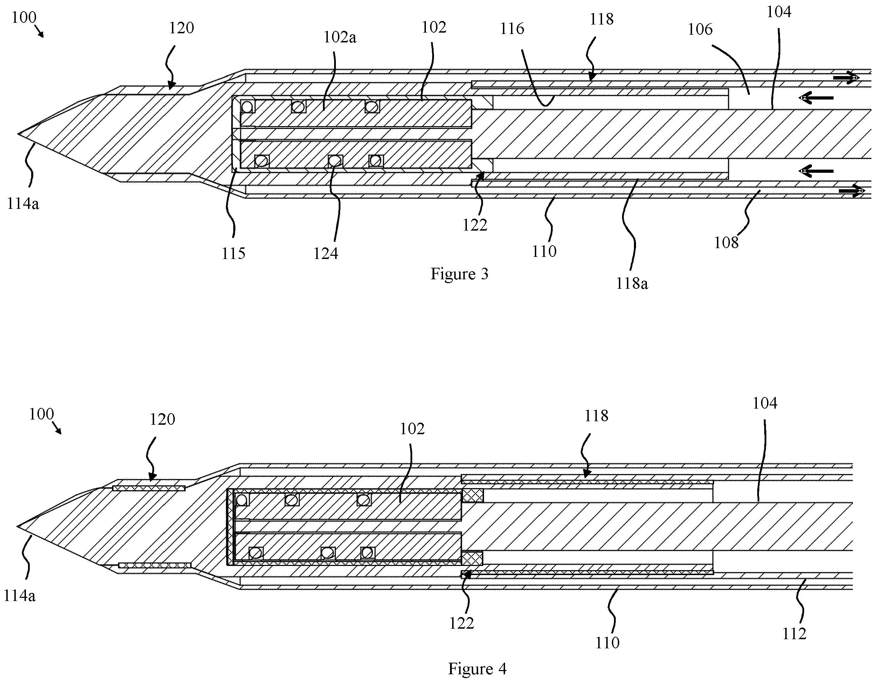

An exploded and a sectional view of the distal end of the ablation probe 100 are shown in Figures 2 and 3 respectively.

The ablation probe 100 comprises an applicator 102 arranged to apply radiation to heat surrounding tissue. The applied radiation may be adapted to cause localised heating and destruction of malignant cells around or near to the applicator 102. The applicator 102 may be arranged to apply any suitable form of radiation to surrounding tissue such that the desired heating is caused. The applicator 102 may, for example, be arranged to emit microwave or RF radiation, or may emit any other suitable radiation to cause heating. The applicator 102 is arranged at or near a distal end of the ablation probe 100 so that it can be positioned in a desired position relative to the tissue to be treated. The applicator 102 may be formed from a ceramic material with suitable dielectric properties (for example, zirconia) according to the energy it is arranged to apply.

The ablation probe 100 further comprises a feeding cable 104 which is arranged to supply electromagnetic energy to the applicator 102. Only part of the length of the feeding cable is shown in Figures 2 and 3. The feeding cable 104 may be any elongate member suitable for supplying electromagnetic energy to the applicator (e.g. a conductor). The feeding cable 104 may run along at least part or all of the length of the ablation probe 100 to deliver a supply of energy to the applicator 102. In the described embodiment, a distal end of the feeding cable 104 is coupled to a proximal end of the applicator 102 and a proximal end of the feeding cable 104 (not shown in Figures 2 and 3) is coupled to a generation means (also not shown in the Figures) suitable for generating the desired signal to supply energy to the applicator 102.

The ablation probe further comprises a coolant circuit flow path forming a coolant supply circuit. The flow of coolant is shown by arrows in Figure 3. The coolant circuit flow path comprises a first coolant path 106. In the described embodiment, the first coolant path is a coolant delivery path via which coolant is able to flow in a direction towards the applicator 102. For example, the coolant delivery path 106 may deliver a flow of coolant towards the distal end of the ablation probe 100 from a coolant supply means (not shown in the Figures) coupled to the coolant delivery path 106 at the proximal end of the ablation probe 100. The flow of coolant may help control the temperature of the ablation probe 100 during use. This may allow energy to be delivered to the surrounding tissue for an extended period of time without the ablation probe 100 overheating and being damaged, or causing injury to healthy tissue. The coolant may be a fluid, and may be water, saline solution, a cryogenic gas or any other suitable coolant known in the art.

The coolant circuit flow path further comprises a second coolant path 108. In the described embodiment, the second coolant path 108 is a coolant return path via which coolant can return from the applicator. The coolant return path 108 may therefore return the supply of coolant from the distal end of the ablation probe 100 to the proximal end.

The ablation probe 100 further comprises a deformable member 110 which is arranged to move between an insertion configuration in which insertion of the ablation probe 100 is facilitated and a deployed configuration (shown in Figure 3). When in the deployed configuration, the coolant return path 108 is provided by the deformable member 110. In some embodiments, no coolant return path may be provided when the deformable member is in the insertion configuration. This may allow the profile of the ablation probe to be minimised. In other embodiments, the return path may not be completely absent when the deformable member is in the insertion configuration. The insertion configuration therefore provides a configuration in which the ablation probe 100 may be suitable for delivery to the desired location within the body. The insertion configuration may, for example, correspond to a suitable size and/or shape adapted to allow insertion with reduced risk of undesired tissue damage. When in the insertion configuration, the ablation probe 100 may, for example, have a low profile (e.g. small cross sectional size) for ease of insertion through tissue without causing injury or insertion through the working channel of an endoscope.

In other embodiments, the first coolant path 106 may act as a coolant return path. In this embodiment, the first coolant path 106 is arranged to carry a flow of coolant away from the applicator. In this embodiment, the second coolant path 108 may act as a coolant delivery path arranged to carry a flow of coolant towards the applicator. A combination of the first and second coolant paths may therefore form a coolant circuit arranged to deliver a flow of coolant towards and away from the applicator, where the coolant can flow in either direction along each of the first and second coolant paths.

The ablation probe 100 may be delivered to the desired location whilst the deformable member 110 is in the insertion configuration. Once at the desired location, the deformable member 110 may be moved to the deployed configuration to allow flow of the coolant away from the applicator 102. The coolant can then flow via the coolant delivery and return paths to cool the ablation probe 100 during use. The deformable member 110 therefore is able to provide an insertion configuration suitable for delivery to the ablation site when the coolant flow is not required. Once the ablation probe is in position, the deformable member 110 may be moved to a configuration suitable to provide a flow of coolant as required during delivery of energy from the applicator 102. When in the deformable member is in the insertion configuration the overall diameter of the ablation probe may be between about 13 to about 25 gauge (approximately 2.5 to 0.5 mm). This may allow easy insertion.

As can be seen in Figures 2 and 3, the coolant return path 110 may be provided only by the deformable member along at least a portion of a length of the ablation probe 100. For example, along at least part of the length of the ablation probe 100, no other channels or conduits to carry returning coolant may be provided in addition to the coolant return path 108 formed by the deformable member 110. This may allow the ablation probe 100 to have a small cross sectional size when the deformable member is in the insertion configuration.

In some embodiments, the deformable member may not be provided. In such embodiments, a non-deformable tubular member or similar suitable component may be provided in which to contain the coolant return path 110. In other embodiments, other suitable conduits may be provided to carry coolant along the length of the ablation probe within or outside the tubular member. The coolant flow paths may be provided by conduits within the feeding cable itself, or separate tubes running inside or outside of the tubular member 112.

The ablation probe further comprises a tubular member 112 (e.g. a hypotube, or braid / coil reinforced tubing) arranged to house at least part of the length of the feeding cable 104. The tubular member 112 may be formed from a metal material which has sufficient rigidity to allow the ablation probe to be inserted into tissue. In other embodiments, the tubular member 112 may be formed from any other suitable material, and may be formed from a superelastic material, for example Nitinol.

In other embodiments, the tubular member 112 may be formed form an elastic material (and not specifically a superelastic material). By forming the tube from an elastic (or superelastic) material it may withstand permanent deformation after being delivered through the tortuous path of a working channel. As the ablation probe extends from the working channel it may consequently follow a straight path, rather than following a curved path caused by the material being deformed by the shape of the working channel. This may help to more easily guide the distal tip of the ablation probe to the desired position.

In the presently described embodiment, the coolant delivery path 106 is provided by a channel formed between the feeding cable 104 and inside wall of the tubular member 112. For example, clearance between the feeding cable 104 and the inside wall of the tubular member 112 may provide space for coolant to flow. In other embodiments, slots may be cut into the inside wall of the tubular member 112 to provide a space through which coolant can flow. The amount of clearance may be specified to ensure an adequate flow of cooling is achieved while maximising the power carrying capacity of the feeding cable.

The tubular member 112 may comprise one or more holes 112a through which coolant is able to flow between the first part of the coolant circuit flow path within the deformable member 110 and the second part of the coolant circuit flow path within the tubular member. The holes 112a may be located at or near the distal end of the tubular member to provide a flow of coolant at or near to the applicator 102.

The deformable member 110 is formed by an inflatable member arranged to move between a deflated configuration when the deformable member 110 is in the insertion configuration and an inflated configuration when the deformable member 110 is in the deployed configuration. The inflatable member may thus form a balloon which may be inflated by the flow of coolant (e.g. the inflatable member may inflate due to the pressure of the coolant). In the described embodiment, the inflatable member has an inside diameter that matches the outside diameter of the tubular member 112 which is surrounds. The inflatable member may inflate to a larger diameter when the cooling system is pressurised. This may therefore form a conduit for the cooling fluid to return from the applicator 102. When moving to the inflated configuration, some, or all, of the inflating member may change shape (e.g. expand) to allow space for the coolant to flow. When the inflation member is deflated, the insertion profile of the ablation probe 100 may be reduced (e.g. minimised) to aid delivery to the target ablation site. When the ablation therapy has been delivered, the inflation member may be deflated so that it returns to its original diameter to facilitate removal.

The ablation probe 100 further comprises a coupling body 114. The coupling body is adapted to structurally couple the applicator, tubular member and deformable member (where provided) together. The coupling body 114 comprises a cavity 116 in which the applicator 104 is at least partly encapsulated. In order to house the applicator, the coupling body 114 may comprise a hole (e.g. an axial bore hole) extending from its proximal end. The hole may be sized to receive the applicator 102. In the present embodiment, all of the applicator 102 is inserted into the cavity 116 so that it is contained within the coupling body 114. The feeding cable 104 extends along the length of the cavity 116, and out of its open end so as to allow a connection to the applicator 102.

The tubular member 112 may be assembled over (or into) the coupling body 114 to align with, and/or be proximal of, the applicator feedpoint. The applicator feedpoint is the point at which the feeding cable connects to the applicator (i.e. where the inner conductor of the feeding cable is inserted into the applicator as will be described later). The applicator 102 may include a counter bored recess, proximally to accommodate the cable outer conductor and ensure an overlap is formed between the applicator 102 and the tubular member 112. The counter bored recess may form the tubular member coupling interface and feeding cable interface as described later.

In some embodiments, the coupling body 114 may be moulded around (e.g. using an insert moulding process) the applicator 102 so that it is encapsulated, and thus reducing the need for adhesives in the assembly. Where the coupling body is moulded in this way it may fit tightly around the applicator so that there is no clearance between them. In other embodiments, a potting compound or adhesive 115 may be provided between the applicator 102 and the coupling body 114. By encapsulating the applicator 102 within the coupling body 114 in this way a secure connection between the applicator 102 and the coupling body 114 may be formed.

The coupling body 114 further comprises a tubular member coupling interface 118 at which the coupling body 114 is coupled to the tubular member 112. The tubular member coupling interface 118 may be located at the proximal end of the coupling body 114. The tubular member coupling interface 118 may take a number of different forms, which may include an overlapping joint between the tubular member 112 and the coupling body 114 as will be described in more detail later. The profile and length of the interface may be optimised to enhance welding, for example providing a lap-joint, butt-joint or a lap-butt hybrid joint. Other types of coupling may however be used. The wall thickness of the overlapping section 118 of the coupling body 114 at the coupling interface 120 may be chosen or varied to create the optimal mechanical response. This could include creating a graded stiffness or strain relief effect between the applicator 102 and the tubular member 112.

The coupling body 114 further comprises a deformable member coupling interface 120 at which the coupling body 114 is coupled to the deformable member 110. The deformable member coupling interface 120 may take different forms depending on the materials used to form the deformable member 110 and the coupling body as will be described in more detail later. The coupling interface may, for example, comprise an adhesive or welded joint. The profile and length of the interface may be optimised to enhance welding, for example providing a lap-joint, butt-joint or a lap-butt hybrid joint. Other types of coupling may however be used.

The coupling body 114 further comprises a pointed distal tip 114a adapted for piercing tissue. The pointed distal tip of the coupling body 114 forms the distal tip of the ablation probe 100 and may have any suitable shape for piecing tissue. It may, for example, be a three sided trocar shape, conical shape or bevel cut. It may be formed by a moulding, grinding or ablation process.

The coupling body 114 acts to encapsulate the applicator 104 and provide a secure coupling to the deformable member 110 and the tubular member 112. The material from which the applicator is formed (e.g. ceramic) may be difficult to machine and bond to other components formed from different materials. By encapsulating the applicator a direct connection between the applicator and the deformable member and/or tubular member is not required. The indirect coupling via the coupling body may aid mechanical strength, while provide desired levels of flexibility and ease of manufacture by reducing complexity.

The portion of the coupling body surrounding the antenna may be suitably thin walled so as not to compromise the operation of the applicator. The portion of the coupling body surrounding the applicator may for example have a wall thickness of between 50 µm and 150 µm. In one embodiment it may be 80 µm. The dielectric permittivity and the relative wall thickness of the coupling body where it overlaps with the applicator are important to control power delivery performance.

A coupling interface 122 between the feeding cable 104 and the coupling body 114 may also be provided. This may aid the strength of the coupling between the applicator 102, feeding cable 104 and coupling body 114. The feeding cable coupling interface 122 may take the form of an adhesive (e.g. UV cure or cyanoacrylate adhesive) or potting compound (e.g. an epoxy) between an outer surface of the feeding cable 104, and the interior surface of the cavity 116. In other embodiments, other types of bonding interface between the feeding cable 104 and the coupling body 114 may be provided. In some embodiment, a mechanical lock may be formed between the components to ensure secure coupling. In yet further embodiments, no direct coupling may be provided between the feeding cable and the coupling body 114.

The coupling body 114 may be at least partly formed from a plastics material. This may provide a more favourable alternative material with which to bond the deformable member 110 and the tubular member 112 compared to the ceramic of the applicator 104. In some embodiments, all of the coupling body may be formed from a plastics material.

The coupling interfaces 118, 120, 122 between the coupling body 114 and the deformable member 110, tubular member 112, applicator 104 and feeding cable are shown as cross-hatched patterned regions in Figure 4. The types of bond used may be tailored to the material of the components being bonded and the desired level of strength and flexibility as described below. In some embodiment, a mechanical lock may be formed between the components to ensure secure coupling.

The material used to form the coupling body 114 may be selected to provide the optimum mechanical strength and flexibility, thermal resistance and suitability for processing. The material used to form the coupling body may have any one or more of the properties in the following paragraph:
For example, the material may be chosen with high mechanical toughness (e.g. Charpy notched impact strength in a range between 1 and 50 kJ/m² to ensure it can withstand high strains at the tubular member coupling interface 118. A material may be selected with high hardness (Rockwell Harness (m-Scale) in a range between 10 and 50) or flexural modulus (in a range between 1-50 GPa) to increase the sharpness and tissue piercing performance of the distal tip. The material may be selected based on its thermal resistance (e.g. a heat deflection temperature in a range between 80 and 400 degrees Celsius at 1.8 MPa) to ensure it can withstand heating effects originating from the applicator 104 during therapy delivery (which can exceed 100 degrees Celsius). The material may be selected based on its rheology characteristic and its suitability for moulding of geometries with thin wall sections (e.g. wall sections have a thickness of < 0.15mm, typically 0.08 mm). The coupling body may be formed from a material having a dielectric constant greater than 5, and preferably greater than 20. This may help reduce interference with the functioning of the applicator.

The material chosen for the coupling body may provide an optimum combination of these properties. The coupling body may therefore be formed from glass filled (e.g. 30% glass filled) Nylon 6.

In one embodiment, at least part of the coupling body 114 may be formed from a transparent material such as a transparent plastics material. By forming the coupling body from a transparent material, the joint between the coupling body 114 and the components to which it is bonded may be visible after assembly. This may allow the use of UV curing adhesive to form bonds between components. For example, a region of the coupling body 114 forming any one or more of the coupling interfaces with the deformable member 110, tubular member 112, feeding cable 104 or which surrounds the applicator 102 may be formed from a transparent plastics material. In some embodiments, all of the coupling body 114 may be formed from a transparent plastics material.

The coupling body is formed from nylon or glass reinforced nylon. For example, a region of the coupling body 114 forming any one or more of the coupling interfaces with the deformable member 110, tubular member 112, feeding cable or which surrounds the applicator 104 may be formed from glass reinforced nylon. In some embodiments, all of the coupling body 114 may be formed from glass reinforced nylon.

In order to provide compatibility with the coupling body, the deformable member 110 may be formed from the same or similar material as the coupling body 114. The deformable member and at least a portion of the coupling body forming the deformable member coupling interface may, for example, both be formed from materials having compatible melting temperatures. By compatible, we mean suitable for forming a welded joint i.e. the melting temperature may be equal or similar (e.g. having a difference of 100 degrees (or 50 degrees) or less between them).

For example if the deformable member is formed of Nylon 6, the coupling body may be formed of Nylon 6 also, or glass reinforced Nylon 6. The deformable member coupling interface 120 may then be formed by a welded joint formed by melting of the material forming the coupling body and the deformable member. The welded joint may be formed by a re-flow, laser or ultrasonic welding process, or another suitable process. This may provide an improved joint between them. A similar welding process may be used to join the coupling body 114 and the tubular member 112 if the tubular member is formed from a polymeric material (e.g. Nylon, Pebax, PEEK or any other polymer material).

The coupling body 114 may be formed from a single integral piece. In other words, in may be a single component formed from a continuous piece of the same material. This may provide improved mechanical strength and aid manufacture. In other embodiments, different parts of the coupling body may be formed from different materials. For example, the region of the coupling body surrounding the applicator may be transparent or the region of the coupling body forming the deformable member coupling interface may be formed from glass reinforced nylon to allow a welded joint to be formed. In some embodiments, the portion of the coupling body forming the pointed tip may be formed from a different material to the rest of the coupling body. The pointed tip section maybe formed of a non-polymeric material (e.g. a metal or ceramic) which may have superior tissue piercing properties. In this embodiment the coupling body may include a geometry or interlocking shape to receive or engage with a separate component forming the pointed tip. This may allow the pointed tip component to be fixed securely to the coupling body (e.g. using an adhesive or insert moulding process).

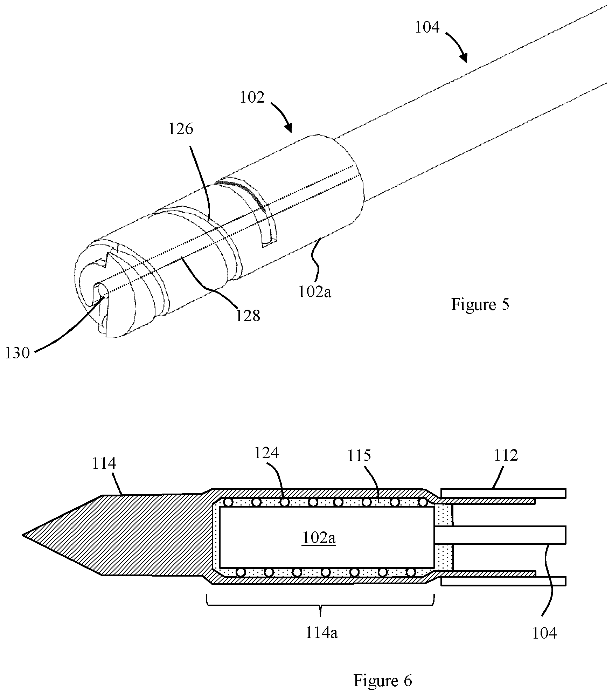

As can be seen illustrated in Figure 2, the applicator 102 comprises an antenna body 102a having a generally cylindrical shape. A close up of the antenna body 102a is also shown in Figure 5, in which only the applicator 102 and the connected part of the feeding cable 104 are shown. The applicator 102 may further comprise an antenna conductor 124. The antenna conductor 124 is formed from an electrically conducting element (e.g. a wire) that is electrically connected, or is part of, the feeding cable 104. **In** the presently described embodiment, the feeding cable 104 comprises an inner conductor 104a, an outer conductor 104b and a dielectric material between them. The antenna conductor 124 is formed from part of the inner conductor 104a of the feeding cable 104 that extends beyond the distal end of the dielectric material and outer conductor 104b. In other embodiments, a separate conducting wire may be coupled to the feeding cable 104 to form the antenna conductor.

Referring again to Figure 2 and particularly Figure 5, the antenna body 102a comprises an outer surface having a channel 126 in which the antenna conductor 124 is received. The channel may extend along a helical path around a central axis of the antenna body 102a to form a helical shaped antenna conductor 124. Other shapes of antenna conductor 124 may be provided by using a correspondingly shaped path around the outer surface of the antenna body. The channel in the antenna body 102a may help maintain the antenna conductor 124 in the desired shape. It may also allow the antenna conductor 124 to fit compactly within the cavity 116 of the coupling body 114. The antenna conductor 124 may, for example, be easier to manufacture compared to the use of conducting material deposited on the surface of the antenna to form the antenna conductor 124.

The antenna conductor 124 may extend through an axial through hole 128 formed in the antenna body 104a. The antenna conductor 124 may extend along the central axis of the applicator body 102a, through the applicator body, and out of a hole 130 at the distal end of the applicator body 102a. The antenna conductor 124 may then extend back along the length of the ablation probe so that it overlaps the portion of its length within the antenna body. This may allow the antenna conductor 124 to form a secure connection with the applicator body 102a. This arrangement may also aid manufacture. For example, the antenna conductor 124 may be provided with a convenient route into and out of the antenna body without requiring a complex passage machined through the antenna body 102a.

In the embodiment shown in Figure 5, the antenna conductor 124 is located within a channel 126 in the applicator body 102a. In an alternative embodiment, illustrated in Figure 6, the antenna conductor 124 is arranged on (e.g. formed onto or wound around) the outer surface of the applicator body 102a. Corresponding reference numbers have been used in Figure 6 for ease of explanation. A potting material 115 or adhesive is provided within the cavity formed between the applicator body 102a and the surrounding inner surface of the coupling body 114. The potting material is arranged to retain the turns of the antenna conductor 124 in the desired shape and position. By positioning the antenna conductor 124 around the outer surface of the applicator body 102a the channels 126 are not required. This aids manufacture, as the material (e.g. ceramic) forming the antenna body 102a may be difficult to machine. In order to accommodate the antenna conductor 124 in this arrangement, the coupling body 114 comprises an expanded or flared portion 114a having an increased diameter in the region where the coupling body 114 surrounds the applicator body 102a. The expanded portion 114a provides a greater clearance between the outer surface of the applicator body 102a and the inner surface of the coupling body 114. This allows sufficient clearance for the turns of the antenna conductor 124, and the potting material 115. In other embodiments, the size of the cavity in which the applicator 102 is housed may be increased without an expanded portion 114a to provide suitable clearance for the antenna conductor 128 by altering the wall thickness of the applicator body 114.

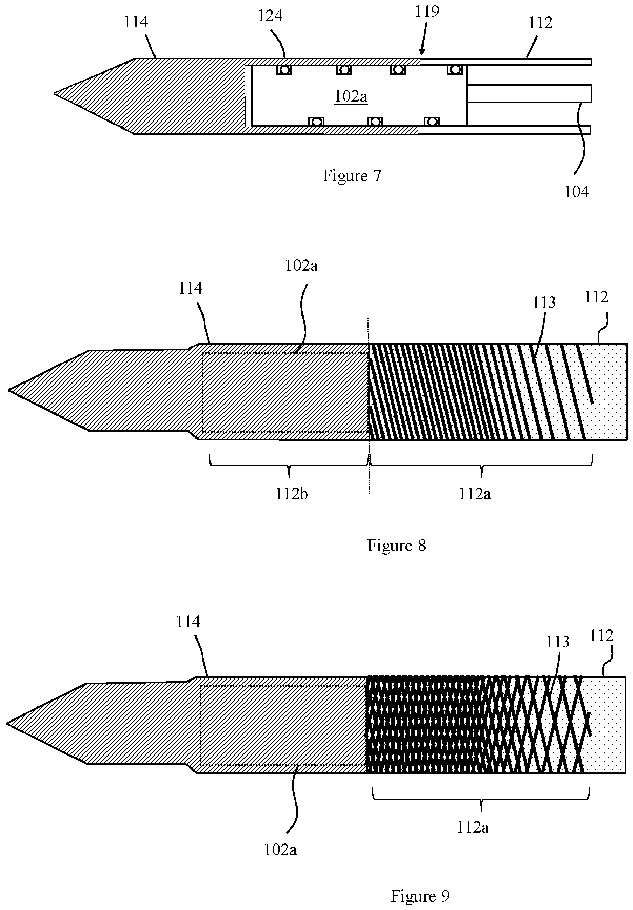

Referring again to Figures 3 and 4, the coupling interface 118 at which the coupling body 114 is coupled to the tubular member comprises an overlapping joint (e.g. a lap joint). The joint is formed from an overlapping portion 118a of the coupling body 114 that extends within the tubular member 112 so that they overlap one another. A bond interface is formed between an outer surface of the overlapping portion 118a of the coupling body 114 and the inner surface of the tubular member 112. This may allow a large connection area and so a secure bond between them. The overlapping portion 118a of the coupling body 114 may have a reduced thickness to form a half lap joint. This may reduce the overall size of the joint between the two components. The bond between the coupling body and the tubular member 112 may be formed by adhesive on the contact surface between the two components. In other embodiments, a welded joint may be used. In the described embodiment, the tubular member 112 fits around the outside of the overlapping portion 118a. In other embodiments the reverse may be the case, with the overlapping portion 118a of the coupling body extending around the tubular member 112. The overlapping portion may help to prevent kinking at the interface between relatively non-flexible applicator and relatively flexible parts of the ablation probe i.e. the tubular member and the feeding cable. A welded or reflow joint may be provided between the tubular member 112 and the coupling body 114. This type of joint may be used for any form of coupling, including the overlapping (lap joint) joints and butt joints described above. An example of a reflowed joint 119 is shown in Figure 7 in which the material forming the coupling member 114 and tubular member 112 is formed into a continuous connection by being welded or reflowed together.

The thickness of the overlapping portion may be in a range between 50 µm and 150 µm. In one embodiment it may be 80 µm. This may provide the desired level of flexibility. In one embodiment, the thickness of the overlapping portion may vary along its length to provide improved control of the stiffness.

Figure 8 illustrates an embodiment in which the tubular member 112 comprises a varying flexibility portion 112a extending along its length from a position adjacent (or axially in line with, axially being along the length of the ablation prove) the proximal end of the applicator body 102a. The varying flexibility portion extends from a boundary with a region of the ablation probe 112b in which the applicator is encapsulated. In the described embodiment, this corresponds to the proximal end of the tubular member 112. The varying flexibility portion 112a defines a section or region of the ablation probe that varies in flexibility along the axial length of the ablation probe. The flexibility varies from a maximum flexibility at the proximal end of the varying flexibility portion 112a (furthest from the applicator) to a minimum flexibility at the distal end of the varying flexibility portion 112a (closest to the applicator). By tapering the flexibility towards the proximal end of the applicator body in this way the risk of kinking of the tubular member 112 is reduced when the ablation probe is bent during insertion. The inventors have found that a smooth transition of flexibility at the edge of the applicator body (i.e. at the boundary between the inflexible region 112b of the ablation probe in which the applicator body 102a is located and the more flexible region of the ablation probe without the applicator body) reduces the risk of kinking.

In the described embodiment, the tubular member 112 is provided with a varying flexibility portion. In other embodiments, any other component extending from a position corresponding to the proximal or distal extent of the region of the ablation probe in which the applicator body is located may have a varying flexibility portion. For example, a region of the coupling body 114 extending distally from a point adjacent or in line with the distal or proximal end of the applicator body 104a may have a similar varying flexibility portion.

In the described embodiment, the varying flexibility portion 112a is formed by varying the amount of a reinforcing material 113 included in the tubular member 112. The tubular member 112 has a reinforcing material 113 applied to its surface in order to provide increased strength and decrease its flexibility. In the embodiment shown in Figure 8, the reinforcing material 113 is formed by a helically coiled fibre or wire applied to the outer surface of the tubular member 112. By gradually decreasing the pitch of the helical coil toward the distal end of the varying flexibility portion 112a (e.g. decreasing the separation between turns of the helix along the length of the ablation probe towards the boundary with the region in which the applicator is located) the overall flexibility of the tubular member 112 may be gradually decreased accordingly.

Other patterns or arrangements of reinforcing material may be used. For example, a braid formed of two overlapping helical coils may be provided as shown in Figure 9. In this case, the fibres forming the braid are closer together at one end of the varying flexibility portion in order to decrease the level of flexibility. In other embodiments, other ways of varying the shape and/or distribution of the reinforcing material may be used. For example, reinforcing rings having relatively smaller axial spacing at the distal end of the varying flexibility portion compared to the distal proximal end may be provided.

In other embodiments, the reinforcing material 113 may be embedded into the tubular member 112 rather than being on the surface. The reinforcing material 113 may be a metal. In some embodiments, the metal may be nitinol. This can provide advantageous shape memory properties. Other metal or suitable reinforcing material can however be used. In yet other embodiments, any other suitable means may be used to vary the flexibility of the material forming the varying flexibility portion along its length. Other types of varying reinforcement may be used, for example, or the material may be varied in density. In some embodiments, a change in flexibility in discrete units or steps rather than a continuous variation may be provided.

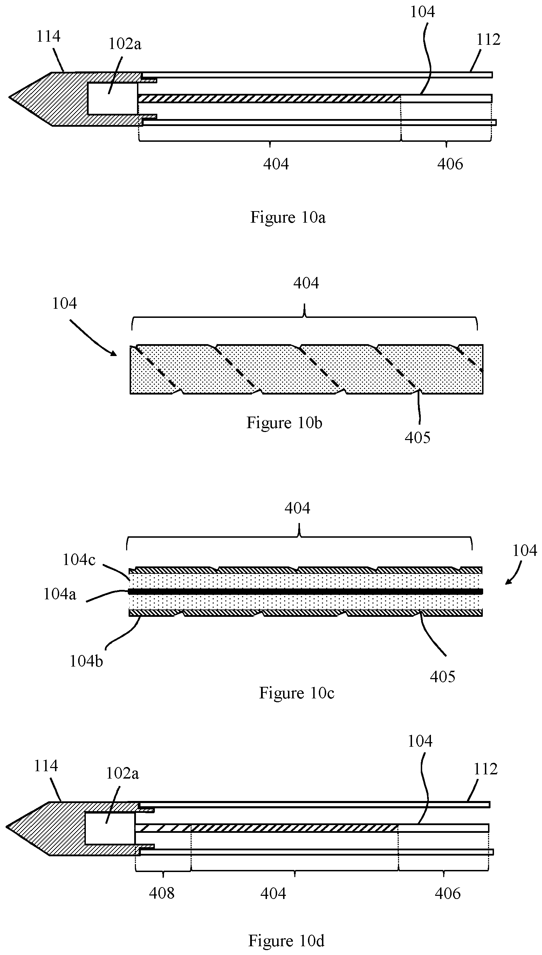

In some embodiments, the flexibility of the feeding cable varies along its length. Referring to Figures 10a to 10c, in one embodiment, the feeding cable 104 comprises a flexibility control portion 404 in a distal region of its overall length (e.g. at or near its distal end (i.e. at or near the coupling between the feeding cable 104 and the applicator body 102a)). The flexibility control portion 404 of the feeding cable 104 comprises a portion of its length having a relatively greater degree of flexibility compared to the adjacent proximal portion 406 (or the rest of the length) of the feeding cable. The flexibility control portion increases the flexibility of the portion of the feeding cable that is inserted into tissue during use. This may be the distal 50-200 mm (or about 100 mm) portion of the overall length of the feeding cable. By increasing the flexibility of the feeding cable in this portion of its length it can more easily return to its original shape after being deflected during tissue insertion. The inventors have found that the increase in the feeding cable flexibility allows the resiliently deformable surrounding tubular member 112 to more easily return the feeding cable to its original shape after it is bent.

The flexibility control portion 404 may be formed by weakening the structure of the feeding cable 104 along the associated part of its length. In the embodiment shown in Figure 10b and Figure 10c, the flexibility control portion 404 is formed by indentations 405 in the outer conductor 104b of the feeding cable 104. The indentation in Figure 10b and 10c is formed by a groove extending along a helical path around the outer surface of the outer conductor 104b. This may allow the indentation to be easily machined in a single cut. In other embodiments, other shapes or patterns of indentation may be used in order to weaken the structure of the feeding cable 104 to the desired degree. In some embodiments, the outer conductor 104b may have slots cut all the way through its thickness to increase flexibility. The skilled person will understand that the slots or indentations formed in the feeding cable 104 are such that the desired level of electrical conductivity and impedance are still provided. In some embodiments, other parts of the feeding cable (e.g. the insulator 104c or inner conductor 104b or other insulating layer) may be modified to provide the increased level of flexibility.

In the embodiment shown in Figure 10a the flexibility control portion 404 of the feeding cable 104 has a constant flexibility along its length (that constant flexibility being greater than the adjacent proximal portion 406 of the feeding cable 104). In other embodiments, the flexibility of the flexibility control portion may vary along its length. In some embodiments, a smooth transition in flexibility is provided along the length of the feeding cable 104. This means that the flexibility varies smoothly over the boundary between the flexibility control portion 404 and the adjacent proximal portion 406 of the feeding cable 104. In other embodiments, a discrete or step-change in flexibility may be provided at the boundary of the flexibility control portion 404 and the proximal portion 406 of the feeding cable. In order to provide a varying level of flexibility the indentations or slots formed in the feeding cable have a varying spacing along its length as described below.

In some embodiments, the feeding cable further comprises a strain relief portion 408 as illustrated in Figure 10d. The strain relief portion extends along the length of the feeding cable 104 from a position at or near to the point of connection with the applicator body 102a. The strain relief portion 408 is formed by a length of feeding cable that has a decrease in flexibility when moving along the length of the feeding cable in the distal direction (i.e. it is stiffer closer to the applicator body). The flexibility of the strain relief portion is lowest at its distal end at or near to the applicator body. The strain relief portion is arranged to reduce the risk of kinking of the feeding cable where it meets the less flexible applicator body by providing a smoother transition between different degrees of flexibility.

The variation of flexibility of the strain relief portion 408 may be created by adding a varying level of reinforcement to the feeding cable so that it is stiffer closer to the applicator body. In the embodiment shown in Figure 10d, the strain relief portion 408 is formed by reducing the weakening of the feeding cable 104 describe above. As shown in Figure 10d, the pitch of the helical indentation in the outer conductor provided in the flexibility control portion 404 is increased along the length of the feeding cable 104 towards the point of connection with the applicator body 102a to form the strain relief portion 408 (i.e. the turns of the helix are further apart closer to the applicator). As the turns of the helical indentation become further apart more material is removed from the feeding cable so as to progressively decrease its flexibility. This allows ease of manufacture of the flexibility control portion 404 and strain relief portion 408. A similar variation in flexibility may be provided along the length of the flexibility control portion 404 to provide the smooth transition with the adjacent proximal portion 406 of the feeding cable. In other embodiments, the flexibility may be varied in other ways. For example, the indentations or slots may be gradually increased in thickness or depth closer to the applicator to increase the overall degree of flexibility of the feeding cable.

Referring again to Figure 1, the ablation probe 100 generally comprises two portions: a needle portion 132 and a catheter portion 134. The needle portion 132 may be arranged at the distal end of the ablation probe 100 and is adapted to be inserted into tissue during use to reach the desired ablation location. The catheter portion 134 may be provided at the proximal end of the ablation probe 100 and is arranged to supply electromagnetic energy and a flow of coolant to and from the needle portion 132. The catheter portion 134 may have an extended length and flexibility for endoscopic use. In other embodiments, a shorter, more rigid catheter portion 134 may be provided for percutaneous use.

In some embodiments, the needle portion 132 may form a small part of the overall length of the ablation probe. For example, the needle portion may be 5 mm to 2000 mm in length, and preferably may be around 70 mm in length. The length of the needle portion 132 may be chosen according to the anatomy to be accessed. For example, the needle portion may be approximately between 10 and 100 mm long for delivery of therapy to organs including the pancreas, or lung, or longer (for example 100-400mm in length) for delivery of therapy percutaneously. A longer length of needle portion may be more suitable for accessing parts of the lung, for example. The catheter portion may be around 1000 mm to 2000 mm in length, and preferably around 1400 mm in length. The length of the catheter portion may be chosen according to the position of the ablation site which must be reached.

**In** other embodiments, the needle portion 132 of the ablation probe (e.g. that having the deformable member) may form a greater proportion of the length of the ablation probe. **In** some embodiments, the entire length of the ablation probe may be formed by the needle portion 132 (i.e. such that a separately defined catheter portion is absent). **In** such an embodiment, the deformable member **110,** which is provided in the needle portion 132, may extend along the majority or all of the length of the ablation probe. **In** such an embodiment, the catheter portion may not be required. For example, if the ablation probe is to be used percutaneously the catheter portion 134 may be shorter than for endoscopic use, or may not be required.

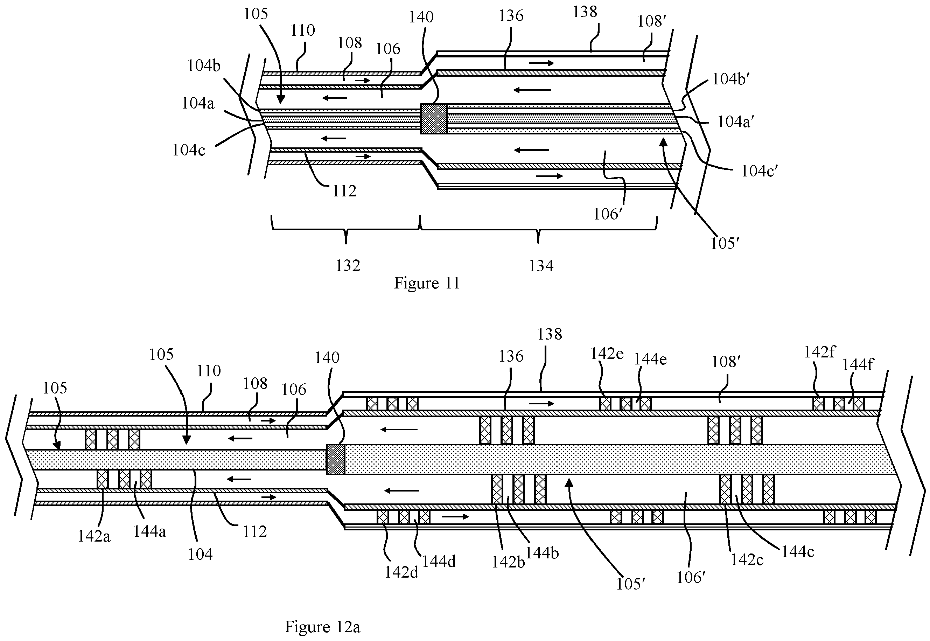

The junction between the needle portion 132 and the catheter portion 134 is shown in Figure 11. The needle portion 132 comprises the deformable member 110, the applicator 102, a distal portion 105 of the feeding cable, a distal portion of the coolant delivery path 106, and the coolant return path 108 (provided within the deformable member). The catheter portion 134 comprises a proximal portion 105' of the feeding cable, a proximal portion 106' of the coolant delivery path, and a catheter portion (or second) coolant return path 108'. The proximal portion of the coolant delivery path 106' is formed by a space between a first or inner catheter tube 136 housing the proximal portion 105' of the feeding cable and the proximal portion of the feeding cable 104. The second coolant return path 108' is formed by the space between the first catheter tube 136 and a surrounding second or outer catheter tube 138. Both of the first and second catheter tubes 136, 138 may be non-deformable, in contrast to the collapsible coolant delivery path provided within the needle portion 132. In other embodiments, any other suitable arrangement of channels or conduits may be provided to form the coolant return and coolant delivery paths within the catheter portion 134.

The greatest cross sectional size of the needle portion 132 may be less than the greatest cross sectional size of the catheter portion 134 (when the deformable member is in the insertion configuration). In other words, the cross section size (e.g. diameter) of the needle portion 132 at its largest point may be less that the cross sectional size (e.g. diameter) of the catheter portion 134 at its greatest point. This may allow the needle portion to access an ablation site whilst reducing any potential for tissue damage. The catheter portion on the other hand may be sized to fit through the working channel of the device with which it is used.

In the described embodiment, the feeding cable 104 is formed by two lengths of cable (the distal portion 105 and the proximal portion 105') joined at the boundary between the needle portion 132 and the catheter portion 134.

The feeding cable 104 may be formed by two lengths of coaxial cable to form an electrical circuit to deliver electromagnetic energy to the applicator 102. The distal portion 105 comprises the inner conductor 104a, outer conductor 104b and dielectric 104c already described. The proximal portion 105' similarly comprises an inner conductor 104a', an outer conductor 104b' and a dielectric 104c'.

In other embodiments a single feeding cable may be used having regions of different thickness to form the distal and proximal portions. In other embodiments, any other suitable conductor may be provided to deliver a supply of suitable electromagnetic energy to the applicator 102. The ablation probe 100 may further comprise a connector 140 arranged to mechanically and electrically connect the distal portion of the feeding cable 105 to the proximal portion of the feeding cable 105'. The connector 140 may connect the different portions of the feeding cable while maintaining an effective impedance match, minimising electrical losses and ensuring a compact configuration of the ablation probe 100.

In the described embodiment, the distal portion of the feeding cable 105 has a corresponding distal cross sectional size, and a proximal portion of the feeding cable 105' has a corresponding proximal cross sectional size, wherein the distal cross sectional size is less than the proximal cross sectional size. The size (e.g. diameter) of the conductor is therefore optimised based on its position within the ablation probe 100. The cross sectional sizes may be chosen to optimise (e.g. maximise) the feeding cable power handling, while also reducing electrical losses and optimising the mechanical strength of the ablation probe 100. In other words, the length of the smaller cross section portion of the feeding cable is minimised by connecting it to a larger cross section feeding cable (e.g. a more efficient cable) for the portion of the ablation probe 100 outside of the needle portion 132. This part of the ablation probe 100 does not need to be inserted into tissue so a small profile is not as important. The cross section of the feeding cable in the catheter portion 134 is therefore increased to reduce power loss where a small cross section is less important.

The needle portion of the ablation probe may therefore have a smaller overall cross sectional size compared to the catheter portion. The needle portion is therefore optimised for insertion into tissue, whilst the catheter portion is optimised for power delivery over the long length of a device working channel through which it is inserted. In use, only the needle portion may protrude from the working channel through which the ablation probe is inserted. It is therefore important for the needle portion to have a relatively small cross sectional size to reduce tissue damage. For the catheter portion a relatively larger cross sectional size can be used. Compared to the needle portion, the catheter portion is instead optimised for power delivery along the length of the working channel. In one example, the needle portion, when the deformable member is in the insertion configuration, may have an overall diameter of 1 mm at its largest point. The catheter portion may have an overall diameter of less than 2 mm at its largest point.

In other embodiments, the cross sectional size of the distal and proximal portions of the feeding cable may be the same. In this case, a reduction in overall size of the needle portion compared to the catheter portion may still be provided by the use of the deformable member.

In the described embodiment, the deformable member 110 extends along at least part of the length of the needle portion 132 as shown in the Figures. The deformable member 110 may, for example, extend from at or near the boundary between the needle portion 132 and the catheter portion 134 and end at or near the distal end of the applicator 102 (e.g. where it is coupled by the deformable member coupling interface 120). The coolant may therefore flow through the deformable member 110 along the length of the ablation probe (e.g. a flow of coolant may be provided between an inlet and an outlet of the deformable member, the inlet and outlet being spaced apart along the length of the ablation probe). The deformable member 110 may be fluidly connected to the non-deformable second catheter tube 138 at a boundary between the needle portion 132 and the catheter portion 134. The coolant may therefore flow through the deformable member 110 (when in the deployed configuration) and then through the non-deformable outer catheter tube 138 in the catheter portion 134 to reach the proximal end of the ablation probe 100.

Referring to Figure 12a, in some embodiments, the ablation probe 100 further comprises spacer members 142a-f arranged to space apart the interior walls of components forming the coolant flow paths 106, 106', 108, 108' carrying coolant along the length of the ablation probe. The spacer members 142a-f are each arranged to maintain the spacing between components forming the coolant flow paths 106, 106', 108, 108'. The spacer members 142a-f each comprise one or more passages 144a-f through which coolant can flow so as to allow adequate coolant flowrate.

In the described embodiment, a set of inner spacer members 142a, 142b, 142c are disposed between the inner surface of the inner catheter tube 136 (or the tubular member 112 depending on their respective position along the length of the ablation probe) and the outer surface of the feeding cable 104. A set of outer spacer members 142d, 142e, 142f are also provided in the space between the outer surface of the first catheter tube 136 and the inner surface of the surrounding second or outer catheter tube 138. Any number of spacer members in each of the inner and outer set may be provided. The three inner spacer members 142a, 142b, 142c and three outer spacer members 142d, 142e, 142f are shown as an example only. In some embodiments, only one of the inner or outer set of spacer members may be provided, with the other set absent. In yet other embodiments, spacer members may be provided between any other components that define a coolant flow path with the arrangement shown in Figure 12a being one example.

The spacer members 142a-f extend between the respective surfaces defining the coolant flow paths and are formed from a relatively incompressible structure or material to maintain the separation of the walls of the coolant flow paths. The spacer members 142a-f therefore help to prevent deformation (e.g. kinking or ovalization) in the shape of the coolant flow paths 106, 106', 108, 108' resulting from flexing of the ablation probe during use. By reducing the risk of deformation of the coolant flow paths the flow of coolant is not affected by flexing of the ablation probe when inserted through tortuous anatomy. The spacer members 142a-f may be provided at discrete positions along the length of the ablation provide as shown in Figure 12a. In the described embodiment, the spacer members 142a-f are provided at equal intervals along the length of the ablation probe. In other embodiments, the spacer members 142a-f may be provided at any suitable positions, for example, where collapsing of the coolant flow paths is more likely. In yet other embodiments, a continuous spacer element may be provided along substantially all of the length of the coolant flow paths.

Referring again to Figure 12a, the channels forming the coolant flow paths 106, 106', 108, 108' are generally annular in cross section. Each of the spacer members 142a-f is formed from a helical shaped component to maintain the annular shape of the coolant channels. The spacer members 142a-f each have turns extending around the central longitudinal axis of the ablation probe. The inner diameter of the helical spacer members 142a-f corresponds to the diameter of the outer surface of the feeding cable 114, tubular member 112 or inner tubular member 136 depending on the respective location of the spacer member (i.e. the inner diameter of the respective coolant conduit). The outer diameter of the helical spacer members 142a-f corresponds to the diameter of the inner surface of the tubular member 112 or outer tubular member 138, again depending on the location of the spacer member (i.e. the outer diameter of the respective coolant conduit). Each of the helical spacer members 142a-f define a respective helical passage 144a-f through which coolant can flow to maintain flow through the coolant flow path in which they are located. The inventors have found that by shaping the spacer members 142a-f in this way kinking of the tubular members forming the ablation probe can be reduced while still allowing easy flexing of the ablation probe. The helical shape aids flexing of the spacer members, whilst still allowing adequate coolant to flow.

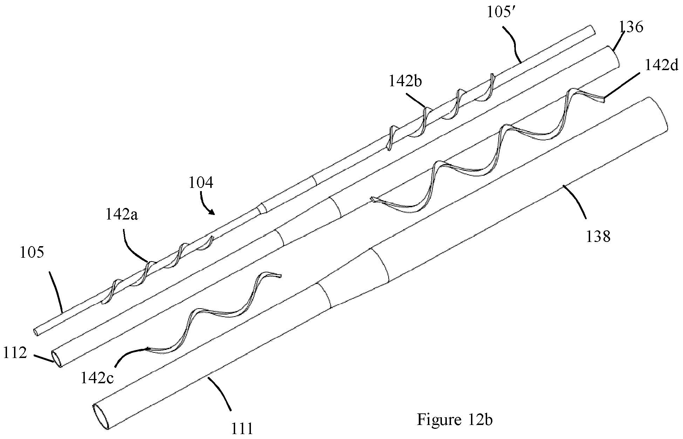

Another embodiment of the ablation probe that includes helical shaped spacer members is illustrated in Figure 12b. In this embodiment, the distal portion of the feeding cable 105 is spaced apart from the tubular member 112 by a spacer member 142a. The proximal portion of the feeding cable 105' is spaced apart from the first catheter tube 136 by a spacer member 142b. The tubular member 112 is spaced apart from a tube 111 (in place of the deformable member 110 in Figure 12a) by a spacer member 142c. The first inner catheter tube is spaced apart from the second outer catheter tube 138 by a spacer member 142d. The exploded view of Figure 12b more clearly shows the helical shape of the spacer members having turns around a longitudinal axis of the ablation probe.

In other embodiments, other shaped spacer members may be provided. The spacer members may, for example, be formed from annular shaped components having axial passages to allow coolant flow. In other embodiments, the spacer members may have an annular sector shape, rather than being formed by a complete annulus. In some embodiments, each of the spacer members may have the same shape. In other embodiments, the shape of the spacer members may vary, for example according to their respective position along the length of the ablation probe.

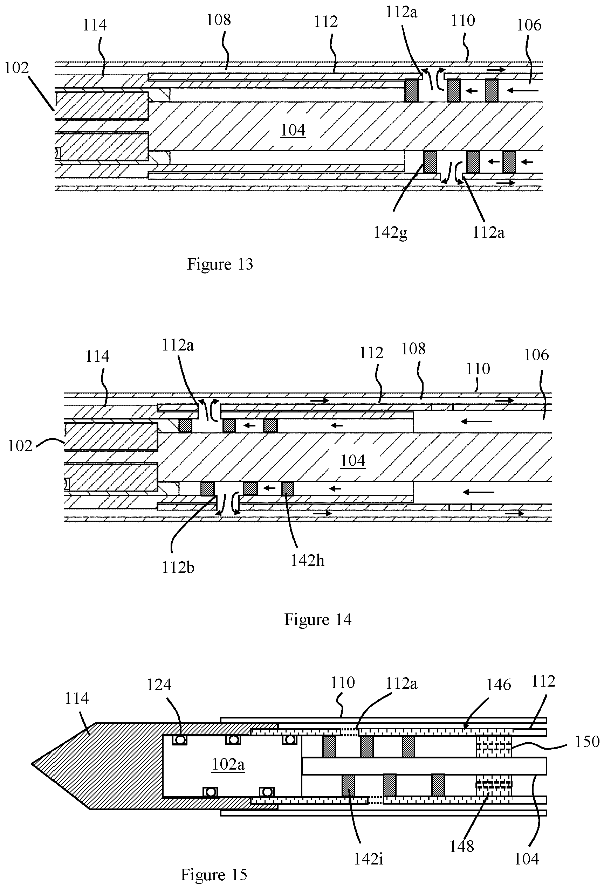

In some embodiments, a spacer member 142g is located at a position where apertures or holes 112a are provided in the walls of a coolant flow channel, the holes allow being to allow flow between coolant channels. An example of this is illustrated in Figure 13. In this embodiment, holes 112a are provided in the tubular member 112 to allow coolant to flow between two separate coolant channels forming the coolant supply circuit (i.e. flow between the first and second coolant flow paths 106, 108 shown in Figure 3). The holes 112a allow coolant to flow between the coolant channels defined between: the feeding cable 104 and inner surface of the tubular member 112; and the outer surface of the tubular member 112 and inner surface of the deformable member 110. The spacer member 142g shown in Figure 13 overlaps the position of the holes 112a. The inventors have found that the channels for coolant created by structures such as the holes 112a in the tubular member 112 cause a structural weakening that makes the ablation probe susceptible to kinking at that point. This may lead to deformation of the coolant flow paths, and reduction in coolant flow. By locating a spacer member at this point the inventors have found that the risk of deformation of the coolant flow path in this way can be alleviated.

A spacer member may be provided at any position where coolant flows between coolant flow paths through slots or holes that cause a weakening in the walls forming the coolant flow paths. In the embodiment shown in Figure 14, aligned holes 112a, 112b are formed in the tubular member 112 and the applicator body 114 respectively to allow coolant to flow between the first and second coolant flow paths. A spacer member 142h is provided at the position of these slots between the outer surface of the feeding cable 104 and the inner surface of the applicator body 114. In embodiments where the deformable member 110 is replaced by a rigid tubular member, similar spacer members may be provided in the second flow path 108 at the position of the holes 112a, 112b.

The spacer member shown in Figures 13 and 14 may be similar to those described in connection with Figure 12a or 12b. It may therefore have a helical shape to allow support of the coolant flow path while allowing coolant to flow and overall flexibility.

In some embodiments, the ablation probe comprises a choke 146 formed by an electrically conducting region of the ablation probe that is spaced apart from and extends around the feed cable proximal to its connection to the applicator body 102a, and which is electrically connected to the outer conductor of the feeding cable 104 at a point spaced apart proximally from the connection point with the applicator body 102a. An example of such a choke is shown in Figure 15 as the shaded region 146. In this embodiment, the choke is formed from a metallic pocked made up of the outer conductor of the feeding cable, a surrounding conducting portion of the feeding cable 112 and an electrically connecting base member 148. The base member 148 electrically connects the tubular member 112 and the outer conductor of the feeding cable 104 across the first coolant flow path at a point spaced apart proximally from the applicator. The base member 148 comprises passages 150 to allow coolant to flow. In this embodiment, a spacer member 142i is located within the choke 146. The spacer member 142i is similar to those already described above, and also has a helical shape. In this embodiment, the spacer member 142i is formed from an electrically insulating material. This ensures that it does not interfere with the functioning of the choke 146. Where the spacer members described herein are located outside of the choke they may be formed from either an electrically insulating or conducting material.

The spacer members defined above are described in use with an ablation probe comprising a coupling body 114. The spacer members may however be used with any ablation probe having a similar arrangement of coolant channels as shown in Figures 12a or 12b. The spacer members may be used in combination with other ablation probes, for example those in which the deformable member and/or the coupling body described herein may be absent.

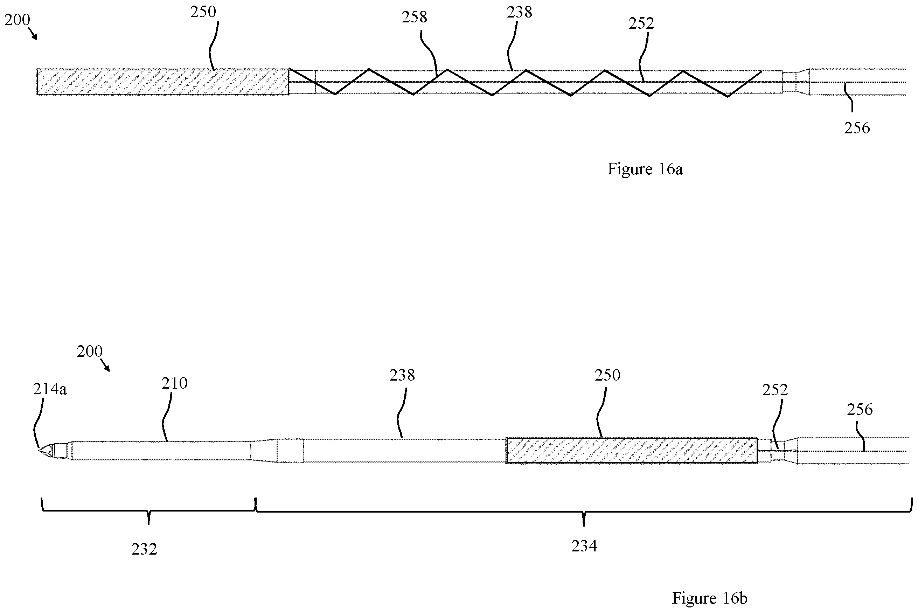

Another embodiment of an ablation probe 200 is shown in Figures 16a to 18b. The ablation probe illustrated in Figures 16a to 18b has corresponding features to the embodiments already described. Corresponding reference numbers have been used accordingly. The ablation probe 200 comprises a needle portion 232 and a catheter portion 234. As described above, the needle portion 232 comprises a deformable member 210, applicator, a distal portion of the feeding cable, a distal portion of the coolant delivery path, and the coolant return path (provided within the deformable member). The catheter portion comprises a proximal portion of the feeding cable, a proximal portion of the coolant delivery path, and a catheter (or second) coolant return path. The proximal portion of the coolant delivery path is formed by a space between a first or inner catheter tube housing the proximal portion of the feeding cable and the feeding cable. The catheter coolant return path is formed by the space between the first catheter tube and a surrounding second or outer catheter tube 238.

The ablation probe 200 comprises a sheath member 250. The sheath member 250 is movable between a first position (i.e. a sheathed position) in which it surrounds the pointed tip 214a of the coupling body 214 and a second position (i.e. an unsheathed position) in which the pointed tip 214a is uncovered or exposed by the sheath member 250. Figure 16a shows the sheath member 250 in the first position, with the second position illustrated in Figure 16b. The sheath member 250 may move the length of the ablation probe between the first and second positions. A movement in a proximal direction provides movement from the first to the second position, with distal movement providing movement from the second to the first positions. The sheath may therefore overlap the sharp tip of the ablation probe during delivery of the device through the working channel of an endoscope or similar device. This may prevent damage to the inner surface of the endoscope (or navigation system) working channel caused by the sharp tip. By moving the sheath member 250 to the second position it may be exposed after the pointed tip 214a has exited the working channel (e.g. of the ENB system) to enable the device to pierce into the target lesion.

The sheath member 250 is formed from a tubular member that fits around or surrounds the body of the ablation probe. The sheath member may form an outer layer of the ablation probe so as to form an outer protective barrier between the ablation probe and the working channel of a delivery device with which it is used. The sheath member 250 may be formed from a material suitable to withstand piercing by the pointed tip 214a, but still have the desired flexibility to allow delivery of the ablation probe along a tortuous route. The sheath member 150 may, for example, be formed from a plastics material (e.g. Pebax or Nylon) or a braid / coil reinforced polymer tube.

As can be seen in Figure 16a and 16b the sheath member 250 extends only part way along the length of the ablation probe 200 between its distal and proximal ends. The ablation probe 200 further comprises one or more fixing wires or control lines 252 connected to the sheath member 250. The control lines may be elongate wires or cables running the length of the ablation probe. They may, for example, be described as pull wires. The fixing wires extend along the length of the ablation probe between the sheath member 250 and a position at or near to the proximal end of the ablation probe 200. The fixing wires are adapted to allow control of the position of the sheath member 250 and facilitate movement between the sheathed and unsheathed positions. The fixing wires may be connected to a portion of the handle that connects directly to endoscope or bronchoscope, ENB system or other delivery system. This ensures it cannot move distally relative to the delivery system. The handle may have two parts that are movable relative to each other: a first part may be coupled to the ablation probe and the second part coupled to the delivery system (fixed relative to the working channel) The ablation probe 200 is connected to the moveable element (i.e. first part) of the handle allowing it to be advance forward relative to the sheath, into the target lesion.

The fixing wires 252 may be formed by any suitable elongate members. They may, for example, be formed from metal or plastic wires. The control lines may be formed from rods or similar structures along some or all of their length. The rods may be configured not to buckle if handle system is used to move the sheath 250 distally over the applicator 102.

By providing a sheath member 250 that extends over only part of the length of the ablation probe the space that it takes up may be reduced. This allows a more compact arrangement, which may be particularly advantageous when the ablation probe is used with an endoscope or similar device where space inside the working channel is limited. Part of the length of the sheath member 250 is effectively replaced by the fixing wires 252, rather than extending the sheath all of the way to the proximal end of the device to allow it to be positioned during use. **In** this way, a more compact ablation probe may be provided. More space may be allowed for the coolant flow paths and feeding cable within the size constraint of the working channel in which it is to be inserted.

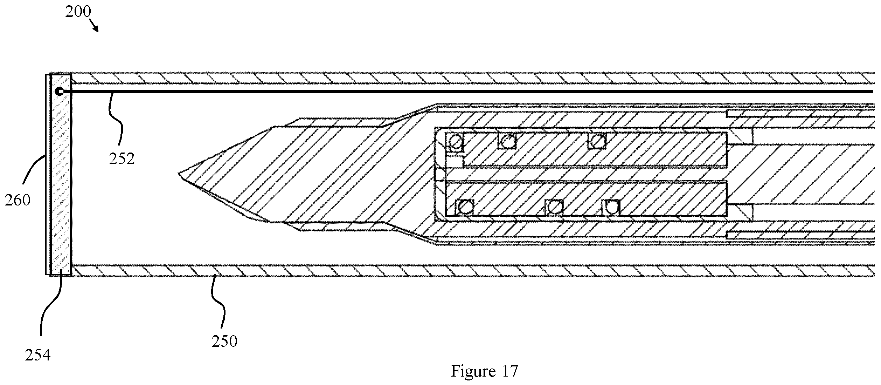

The one or more fixing wires 252 may be connected at or near the distal end of the sheath member 250. This may provide improved control of the movement of the sheath member 250. In one embodiment, the sheath member 250 may comprise a reinforcing ring 254 at its distal end. An example of such an embodiment is shown in Figure 17. In this embodiment, the one or more fixing wires 252 are connected to the reinforcing ring 254. In some embodiments, a plurality of fixing wires may be provided. The plurality of fixing wires may be distributed evenly around the sheath member 152 (e.g. equally around its circumference, or equally around the circumference of the reinforcing ring) so as to apply an even force.

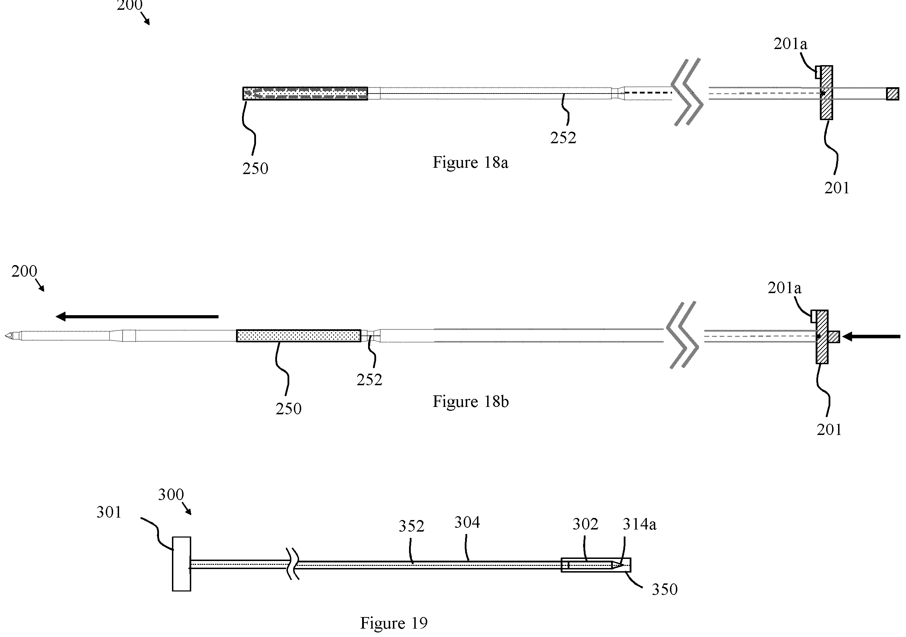

Referring to Figures 18a and 18b the ablation probe is coupled to a handle 201 at or near its proximal end. The ablation probe 200 and handle 201 may together form an ablation probe assembly. The handle 201 is adapted to allow manipulation of the ablation probe during use. The ablation probe is slidably coupled to the handle 201 so that it can be moved relative to the handle between an extended and retracted position (shown by the arrows in Figure 18b). The retracted position is shown in Figure 18a, with the extended position shown in Figure 18b. The handle portion 102 comprises a connecting mechanism 201a with which the handle 201 is connectable to a delivery device (e.g. an endoscope) with which the ablation probe 200 is to be used. When connected, the handle 201 may be fixed relative to the working channel of the delivery device. Once fixed in this may, sliding movement of the ablation probe 200 relative to the handle 201 causes sliding movement of the ablation probe 200 along the length of the working channel. This may cause the distal end of the ablation probe 200 to extend from the distal end of the working channel to access tissue when the delivery device has been positioned at the required location within the body.

In the present embodiment, the one or more fixing wires 252 are connected between the handle 201 and the sheath member 250. The fixing wires are arranged to restrict the range of movement of the sheath member 250 in a proximal direction away from the handle 201. The sheath member 250 is therefore restricted from moving along the length of the ablation probe 200 in a proximal direction away from the handle 201 by a maximum distance set by the length of the fixing wires 252. The fixing wires may be substantially inelastic so that they can pull back the sheath. This means that movement of the ablation probe relative to the handle 201 causes movement of the sheath member 250 from the sheathed to the unsheathed configuration. This may allow the ablation probe to be unsheathed using the same action that extends its distal end from the working channel, thus facilitating ease of use.

As already described, the one or more fixing wires 252 extend along the length of the ablation probe. In the described embodiment, the fixing wires 252 extend within a channel 256 or lumen formed in the outer catheter tube of the ablation probe. This may reduce the space taken up by the fixing wires and allow a compact arrangement. The channel or lumen housing the control lines 252 may be formed in the wall of the catheter tube, or in a protrusion on the outer surface of the tube.

The sheath member 250 may form a friction fit with the body of the ablation probe which it surrounds. For example, a friction fit may be formed with the deformable member 210 or any section of the catheter (e.g. any other suitable outer part of the ablation probe away from the deformable member). The friction fit may act to retain the sheath member 250 in the sheathed position during delivery through a tortuous path, until it is retracted using the fixing wires. This may help to keep the sheath member 250 in the sheathed position during insertion into the working channel.

The ablation probe may comprise a biasing member 258 arranged to bias the sheath member 250 towards the sheathed position. The biasing member 258 may bias the sheath member 250 towards distal direction along the length of the ablation probe 200. The biasing member 258 may be a coiled spring arranged around the body of the ablation probe. Other types of biasing member may however be used. The biasing member may be provided in addition or alternatively to the friction fit. The stiffness of the biasing member 258 may be optimised to ensure ease of use. It shall ensure the sharp tip is re-sheathed when withdrawn from the lesion (i.e. provide adequate force to move the sheath from the un-sheathed to sheathed position when required).

The sheath member may comprise a covering member 260 arranged to cover the pointed tip when the sheath member is in the first position. The covering member is adapted to be pierced (i.e. it is frangible) by the pointed tip when the sheath member is moved from the first position to the second position to expose the pointed tip. The covering member may be formed from a membrane covering the distal end of the sheath member as shown in Figure 17. The membrane is pierced by the pointed tip of the ablation probe only when it is exposed from the sheath member. The membrane is pierced by the pointed tip when the control lines pull back and the sheath member slides from the sheathed to the unsheathed positions. The covering member may help to keep the sheath member in place when it travels along the working channel. When the control lines apply force to the sheath, the pointed tip pierces the membrane and the sheath slides to uncover the pointed tip.

In the embodiment shown in Figures 16a to 18b the sheath member 250 is used in combination with the ablation probe described with reference to Figures 1 to 15. The sheath member may however be used in combination with other ablation probes, for example those in which the deformable member and/or the coupling body may be absent.

The sheath member may, for example, be used with any ablation probe having an applicator arranged to apply radiation to heat surrounding tissue; a feeding cable arranged to supply electromagnetic energy to the applicator; and a pointed distal tip adapted for piercing tissue. An example of such an embodiment is shown schematically in Figure 19, which illustrates an ablation probe 300 comprising an applicator 302. The applicator 302 is arranged to apply radiation to heat surrounding tissue as described in connection with other embodiments. The ablation probe 300 comprises a feeding cable 304 arranged to supply electromagnetic energy to the applicator 302. A distal end of the ablation probe comprises a pointed distal tip 314a adapted for piercing tissue. The pointed tip may be provided on the component forming the distal end of the ablation probe, which may be a coupling body as already described, or may be a pointed tip of the applicator or separate tip component. The ablation probe further comprises a sheath member 350 movable between a first position in which it surrounds the pointed tip and a second position in which the pointed tip 314a is uncovered. The sheath member 350 extends part way along the length of the ablation probe between the ablation probe's distal and proximal ends. The ablation probe comprises one or more fixing wires 352 connected to the sheath member. The fixing wires extend along the length of the ablation probe between the sheath member and a position at or near the proximal end of the ablation probe.

The ablation probe 300 may be coupled to a handle 301 as described above in connection with Figures 16a to 18b. The fixing wires 352 may be connected between the sheath member 352 and the handle 301 so that movement relative to the handle causes movement of the sheath member between the sheathed and unsheathed positions. Any of the features described in connection with the embodiments of Figures 16a to 18b can be provided in the embodiment of Figure 19.

Various modifications will be apparent to the skilled person without departing form the scope of the claims. Any feature disclosed in connection with one embodiment may be used in combination with the features of another embodiment.

For the sake of completeness, it is also stated that the term "comprising" does not exclude other elements or steps, the term "a" or "an" does not exclude a plurality, a single processor or other unit may fulfil the functions of several means recited in the claims and any reference signs in the claims shall not be construed as limiting the scope of the claims.

## Claims

1. An ablation probe (100, 200), comprising:
an applicator (102) arranged to apply radiation to heat surrounding tissue;
a feeding cable (104) arranged to supply electromagnetic energy to the applicator (102);
a coolant flow path (106, 108) forming a coolant supply circuit;
a tubular member (112) housing at least part of the feeding cable (104);
and a coupling body (114), the coupling body (114) comprising:
a cavity (116) in which the applicator (102) is at least partly encapsulated;
a coupling interface (118) at which the coupling body (114) is coupled to the tubular member (112);
and a pointed distal tip (114a) adapted for piercing tissue, the coupling body (114) being adapted to structurally couple the applicator (102) and the tubular member (112) together, wherein the coupling body (114) is formed from nylon or glass reinforced nylon, and wherein:
i) the tubular member (112) is formed at least partly from a polymeric material;
and/or
ii) the ablation probe further comprises a deformable member (110, 210) surrounding at least part of the tubular member (112), the deformable member (110, 210) being arranged to move between an insertion configuration and a deployed configuration, and wherein a part of the coolant path (106, 108) is defined between the deformable member (110, 210) and the tubular member (112) when the deformable member is in the deployed configuration, and wherein the coupling body (114) comprises a coupling interface (120) at which the coupling body (114) is coupled to the deformable member (110), the deformable member (110, 210) being formed at least partly from nylon.

2. An ablation probe according to claim 1, wherein any one or more of:
b) the coupling body (114) is formed at least partly from a material having a Charpy notched impact strength in the range between 1 and 50 kJ/m²;
c) the coupling body (114) is formed at least partly from a material having a Rockwell Harness (m-Scale) in the range between 10 and 50;
d) the coupling body (114) is formed at least partly from a material having a flexural modulus in the range between 1-50 GPa;
e) the coupling body (114) is formed at least partly from a material having a heat deflection temperature in the range between 80 and 400 degrees Celsius at 1.8 MPa;
f) the coupling body (114) is formed at least partly from a transparent material;
g) when the ablation probe comprises the deformable member (110, 210), the deformable member (110, 210) and at least a portion of the coupling body (114) forming the coupling interface (120) at which the coupling body (114) is coupled to the deformable member (110, 210) are both formed from materials compatible for thermal welding; and
h) wherein the coupling body (114) is formed from a material having a dielectric constant greater than 5, and preferably greater than 20.

3. An ablation probe according to claim 1 or claim 2, wherein the applicator (102) comprises an applicator body (102a) and an antenna conductor (124), wherein the applicator body (102a) comprises an outer surface having a channel (126) in which the antenna conductor (124) is received.

4. An ablation probe according to claim 3, wherein the channel (126) extends along a helical path around a central axis of the applicator body (102a).

5. An ablation probe according to claim 1 or claim 2, wherein the applicator (102) comprises an applicator body (102a) and an antenna conductor (124), wherein the antenna conductor (124) is arranged on an outer surface of the applicator body (102a) and wherein a potting material or adhesive (115) is provided to secure the antenna conductor (124) in position, and optionally wherein the antenna conductor (124) extends along a helical path around the outer surface of the applicator (102).

6. An ablation probe according to claim 3 or claim 4 or claim 5, wherein the feeding cable (104) comprises an inner conductor (104a), an outer conductor (104b) and a dielectric material between them, and wherein the antenna conductor (124) is formed from part of the inner conductor (104a) of the feeding cable (104).

7. An ablation probe according to claim 6, wherein part of the length of the antenna conductor (124) extends in a distal direction from a distal end of the outer conductor (104b) and within an axial through hole (130) formed in the antenna body (102a).

8. An ablation probe according to any preceding claim, featuring any one or more of:
a) the coupling body (114) is insert moulded around the applicator (104);
b) the coupling interface (118) at which the coupling body (114) is coupled to the tubular member (112) comprises an overlapping portion (118a) of the coupling body (114) that extends within or around the tubular member (112) so that they overlap one another;
c) a welded or reflowed joint is formed between the coupling body (114) and the tubular member (112);
d) a part of the coolant flow path (106, 108) is defined by a space between the feeding cable (104) and the tubular member (112);
e) the feeding cable (104) comprises a strain relief portion (408) extending along the length of the feeding cable (104) from a position at or near to the point of connection between the feeding cable (104) and applicator (102), wherein the strain relief portion (408) is formed by a length of the feeding cable (104) that decreases in flexibility when moving along the length of the feeding cable (104) in the distal direction towards the applicator (102); and/or
f) a, or the, potting compound or adhesive (115) is provided between the applicator (102) and the coupling body (114).

9. An ablation probe according to any preceding claim, further comprising a sheath member (250) movable between a first position in which it surrounds the pointed tip (114a, 214a) of the coupling body (114) and a second position in which the pointed tip (114a, 214a) is uncovered.

10. An ablation probe according to claim 9, wherein the sheath member (250) extends part way along the length of the ablation probe between the distal and proximal ends of the ablation probe, and wherein the ablation probe comprises one or more control lines (252) connected to the sheath member (250), the control lines (252) extending along the length of the ablation probe between the sheath member (250) and a position at or near the proximal end of the ablation probe,
and optionally the one or more control lines (252) are connected at or near a distal end of the sheath member (250),
and further optionally, the sheath member (250) is formed from a tube have a reinforcing ring (254) at its distal end, wherein the one or more control lines (252) are connected to the reinforcing ring (254).

11. An ablation probe according to claim 10, wherein the ablation probe is slidably coupled to a handle (201) provided at or near its proximal end, and wherein the one or more control lines (252) are connected between the handle (201) and the sheath member (250), and the control lines (252) are arranged to restrict the range of movement of the sheath member (250) in a distal direction away from the handle (201).

12. An ablation probe according to any of claims 9 to 11, wherein any one or more of:
a) when dependent on claim 10, the ablation probe comprises an outer catheter tube in which part of the coolant flow path is contained, and wherein the one or more control lines (252) extend within a channel or lumen formed in the outer catheter tube;
b) the sheath member (250) forms a friction fit with the body of the ablation probe around which it extends and/or comprises a biasing member (258) arranged to bias the sheath member (250) in a distal direction; and/or
c) the sheath member (250) comprises a covering member (260) arranged to cover the pointed tip (114a, 214a) when the sheath member (250) is in the first position, the covering member (260) being pierced by the pointed tip (114a, 214a) when the sheath member (250) is moved from the first position to the second position to expose the pointed tip (114a, 214a).

13. The ablation probe according to any preceding claim, further comprising one or more spacer members (142a-f) each arranged to space apart the interior walls of a channel or channels forming the coolant flow path (106, 108); and optionally wherein one or both of:
a) the channel or channels forming the coolant flow path (106, 108) are generally annular in cross section, and at least part of each of the one or more spacer members (142a-f) is generally helical in shape; and/or
b) the coolant flow path (106, 108) comprises a first coolant path (106) and a second coolant path (108), the first and second coolant paths being fluidly connected by one or more connecting holes (112a) extending through respective walls of the channels forming the first and second coolant flow paths (106, 108); and
one of the one or more spacer members (142a-f) is located at a position overlapping the one or more connecting holes (112a).

14. The ablation probe according to any preceding claim, wherein the feeding cable (104) comprises a portion of its length having a relatively greater flexibility compared to an adjacent proximal portion of its length, the portion of greater flexibility being located in a distal region of the feeding cable (104), and optionally wherein the portion of greater flexibility is formed by a weakened portion of an outer conductor (104b) forming the feeding cable (104), and optionally
wherein the portion of greater flexibility comprises an indentation or slot formed in the outer conductor of the feeding cable (104), the indentation or slot extending along a helical path along the length of the feeding cable (104).

15. The ablation probe according to any preceding claim, wherein:
the applicator extends between a distal end and a proximal end; and
the ablation probe further comprises a varying flexibility portion extending from a position adjacent the distal or proximal end of the applicator (102), the varying flexibility portion comprising a region of the ablation probe that has a minimum degree of flexibility closest to the applicator (104) and which increases in flexibility in a direction extending along the length of the ablation probe away from the applicator (104), and optionally
wherein the varying flexibility portion is formed by a region of the ablation probe comprising a reinforcing material, the reinforcing material arranged to vary in shape and/or distribution to vary the flexibility of the varying flexibility portion, and optionally wherein the reinforcing material comprises a helical fibre or wire having a varying pitch, the pitch decreasing along the length of the ablation probe in a direction towards the applicator.

## Patentansprüche

1. Ablationssonde (100, 200), umfassend:
einen Applikator (102), der dazu angeordnet ist, Strahlung zum Erwärmen von umgebendem Gewebe zu applizieren;
ein Zuleitungskabel (104), das dazu angeordnet ist, dem Applikator (102) elektromagnetische Energie zuzuführen;
einen Kühlmittelflussweg (106, 108), der einen Kühlmittelversorgungskreis bildet;
ein röhrenförmiges Element (112), das mindestens einen Teil des Zuleitungskabels (104) beherbergt;
und einen Kopplungskörper (114), wobei der Kopplungskörper (114) umfasst:
einen Hohlraum (116), in dem der Applikator (102) mindestens teilweise eingekapselt ist;
eine Kopplungsschnittstelle (118), an der der Kopplungskörper (114) mit dem röhrenförmigen Element (112) gekoppelt ist;
und eine spitze distale Spitze (114a), die zum Durchstechen von Gewebe angepasst ist, wobei der Kopplungskörper (114) dazu angepasst ist, den Applikator (102) und das röhrenförmige Element (112) strukturell zusammenzukoppeln, wobei der Kopplungskörper (114) aus Nylon oder glasverstärktem Nylon gebildet ist und wobei:
i) das röhrenförmige Element (112) mindestens teilweise aus einem Polymermaterial gebildet ist; und/oder
ii) die Ablationssonde ferner ein verformbares Element (110, 210) umfasst, das mindestens einen Teil des röhrenförmigen Elements (112) umgibt, wobei das verformbare Element (110, 210) dazu angeordnet ist, sich zwischen einer Einführauslegung und einer eingesetzten Auslegung zu bewegen, und wobei ein Teil des Kühlmittelweges (106, 108) zwischen dem verformbaren Element (110, 210) und dem röhrenförmigen Element (112) definiert ist, wenn das verformbare Element in der eingesetzten Auslegung ist, und wobei der Kopplungskörper (114) eine Kopplungsschnittstelle (120) umfasst, an der der Kopplungskörper (114) mit dem verformbaren Element (110) gekoppelt ist, wobei das verformbare Element (110, 210) mindestens teilweise aus Nylon gebildet ist.

2. Ablationssonde nach Anspruch 1, wobei eins oder mehrere von:
b) der Kopplungskörper (114) mindestens teilweise aus einem Material mit einer Charpy-Kerbschlagzähigkeit im Bereich zwischen 1 und 50 kJ/m² gebildet ist;
c) der Kopplungskörper (114) mindestens teilweise aus einem Material mit einer Rockwell-Härte (Skala M) im Bereich zwischen 10 und 50 gebildet ist;
d) der Kopplungskörper (114) mindestens teilweise aus einem Material mit einem Biegemodul im Bereich zwischen 1-50 GPa gebildet ist;
e) der Kopplungskörper (114) mindestens teilweise aus einem Material mit einer Wärmeformbeständigkeitstemperatur im Bereich zwischen 80 und 400 Grad Celsius bei 1,8 MPa gebildet ist;
f) der Kopplungskörper (114) mindestens teilweise aus einem transparenten Material gebildet ist;
g) wenn die Ablationssonde das verformbare Element (110, 210) umfasst, das verformbare Element (110, 210) und mindestens ein Abschnitt des Kopplungskörpers (114), der die Kopplungsschnittstelle (120) bildet, an der der Kopplungskörper (114) mit dem verformbaren Element (110, 210) gekoppelt ist, beide aus Materialien gebildet sind, die zum Wärmeschweißen kompatibel sind; und
h) wobei der Kopplungskörper (114) aus einem Material mit einer dielektrischen Konstante größer als 5 und vorzugsweise größer als 20 gebildet ist.

3. Ablationssonde nach Anspruch 1 oder Anspruch 2, wobei der Applikator (102) einen Applikatorkörper (102a) und einen Antennenleiter (124) umfasst, wobei der Applikatorkörper (102a) eine Außenfläche mit einem Kanal (126) umfasst, in dem der Antennenleiter (124) aufgenommen ist.

4. Ablationssonde nach Anspruch 3, wobei sich der Kanal (126) entlang eines spiralförmigen Weges rund um eine Mittelachse des Applikatorkörpers (102a) erstreckt.

5. Ablationssonde nach Anspruch 1 oder Anspruch 2, wobei der Applikator (102) einen Applikatorkörper (102a) und einen Antennenleiter (124) umfasst, wobei der Antennenleiter (124) auf einer Außenfläche des Applikatorkörpers (102a) angeordnet ist und wobei ein Vergussmaterial oder Klebemittel (115) bereitgestellt ist, um den Antennenleiter (124) in Position zu befestigen, und wobei sich optional der Antennenleiter (124) entlang eines spiralförmigen Weges um die Außenfläche des Applikators (102) erstreckt.

6. Ablationssonde nach Anspruch 3, Anspruch 4 oder Anspruch 5, wobei das Zuleitungskabel (104) einen Innenleiter (104a), einen Außenleiter (104b) und ein dielektrisches Material dazwischen umfasst und wobei der Antennenleiter (124) aus einem Teil des Innenleiters (104a) des Zuleitungskabels (104) gebildet ist.

7. Ablationssonde nach Anspruch 6, wobei sich ein Teil der Länge des Antennenleiters (124) in eine distale Richtung von einem distalen Ende des Außenleiters (104b) und innerhalb eines in dem Antennenkörper (102a) gebildeten axialen Durchgangslochs (130) erstreckt.

8. Ablationssonde nach einem vorhergehenden Anspruch, **dadurch gekennzeichnet, dass** eins oder mehrere von:
a) der Kopplungskörper (114) um den Applikator (104) einsatzgeformt ist;
b) die Kopplungsschnittstelle (118), an der der Kopplungskörper (114) mit dem röhrenförmigen Element (112) gekoppelt ist, einen überlappenden Abschnitt (118a) des Kopplungskörpers (114) umfasst, der sich innerhalb oder um das röhrenförmige Element (112) erstreckt, so dass sie einander überlappen;
c) eine Schweißstelle oder aufgeschmolzene Stelle zwischen dem Kopplungskörper (114) und dem röhrenförmigen Element (112) gebildet ist;
d) ein Teil des Kühlmittelflussweges (106, 108) von einem Zwischenraum zwischen dem Zuleitungskabel (104) und dem röhrenförmigen Element (112) definiert ist;
e) das Zuleitungskabel (104) einen Zugentlastungsabschnitt (408) umfasst, der sich entlang der Länge des Zuleitungskabels (104) von einer Position an oder in der Nähe des Verbindungspunktes zwischen dem Zuleitungskabel (104) und dem Applikator (102) erstreckt, wobei der Zugentlastungsabschnitt (408) von einer Länge des Zuleitungskabels (104) gebildet wird, das in der Flexibilität abnimmt, wenn es entlang der Länge des Zuleitungskabels (104) in die distale Richtung in Richtung des Applikators (102) bewegt wird; und/oder
f) ein/e oder die/das Vergussmasse oder Klebemittel (115) zwischen dem Applikator (102) und dem Kopplungskörper (114) bereitgestellt ist.

9. Ablationssonde nach einem vorhergehenden Anspruch, ferner umfassend ein Hüllenelement (250), das zwischen einer ersten Position, in der es die spitze Spitze (114a, 214a) des Kopplungskörpers (114) umgibt, und einer zweiten Position, in der die spitze Spitze (114a, 214a) freiliegt, beweglich ist.

10. Ablationssonde nach Anspruch 9, wobei sich das Hüllenelement (250) teilweise entlang der Länge der Ablationssonde zwischen dem distalen und dem proximalen Ende der Ablationssonde erstreckt und wobei die Ablationssonde eine oder mehrere Steuerleitungen (252) umfasst, die mit dem Hüllenelement (250) verbunden sind, wobei sich die Steuerleitungen (252) entlang der Länge der Ablationssonde zwischen dem Hüllenelement (250) und einer Position an oder in der Nähe des proximalen Endes der Ablationssonde erstrecken,
und optional die eine oder die mehreren Steuerleitungen (252) an oder in der Nähe eines distalen Endes des Hüllenelements (250) verbunden sind,
und optional ferner das Hüllenelement (250) aus einer Röhre mit einem Verstärkungsring (254) an ihrem distalen Ende gebildet ist, wobei die eine oder die mehreren Steuerleitungen (252) mit dem Verstärkungsring (254) verbunden sind.

11. Ablationssonde nach Anspruch 10, wobei die Ablationssonde gleitend mit einem Griff (201) verbunden ist, der an oder in der Nähe ihres proximalen Endes bereitgestellt ist, und wobei die eine oder die mehreren Steuerleitungen (252) zwischen dem Griff (201) und dem Hüllenelement (250) verbunden sind, und die Steuerleitungen (252) dazu angeordnet sind, den Bewegungsbereich des Hüllenelements (250) in eine distale Richtung von dem Griff weg (201) zu beschränken.

12. Ablationssonde nach einem der Ansprüche 9 bis 11, wobei eins oder mehrere von:
a) wenn abhängig von Anspruch 10, die Ablationssonde eine äußere Katheterröhre umfasst, in der ein Teil des Kühlmittelflussweges enthalten ist, und wobei sich die eine oder die mehreren Steuerleitungen (252) in einem in der äußeren Katheterröhre gebildeten Kanal oder Lumen erstrecken;
b) das Hüllenelement (250) einen Reibschluss mit dem Körper der Ablationssonde bildet, um den sie sich erstreckt, und/oder ein Vorspannelement (258) umfasst, das dazu angeordnet ist, das Hüllenelement (250) in eine distale Richtung vorzuspannen; und/oder
c) das Hüllenelement (250) ein Abdeckelement (260) umfasst, das dazu angepasst ist, die spitze Spitze (114a, 214a) abzudecken, wenn das Hüllenelement (250) in der ersten Position ist, wobei das Abdeckelement (260) von der spitzen Spitze (114a, 214a) durchstochen wird, wenn das Hüllenelement (250) aus der ersten Position in die zweite Position bewegt wird, um die spitze Spitze (114a, 214a) freizulegen.

13. Ablationssonde nach einem vorhergehenden Anspruch, ferner umfassend ein oder mehrere Abstandselemente (142a-f), die jeweils dazu angeordnet sind, die Innenwände eines Kanals oder von Kanälen, der bzw. die den Kühlmittelflussweg (106, 108) bildet bzw. bilden, zu beabstanden; und wobei optional eins oder beide von:
a) der Kanal oder die Kanäle, der bzw. die den Kühlmittelflussweg (106, 108) bildet bzw. bilden, im Querschnitt im Allgemeinen ringförmig sind und mindestens ein Teil eines jeden des einen oder der mehreren Abstandselemente (142a-f) im Allgemeinen spiralförmig ist; und/oder
b) der Kühlmittelflussweg (106, 108) einen ersten Kühlmittelweg (106) und einen zweiten Kühlmittelweg (108) umfasst, wobei der erste und der zweite Kühlmittelweg durch ein oder mehrere Verbindungslöcher (112a), die sich durch entsprechende Wände der den ersten und den zweiten Kühlmittelflussweg (106, 108) bildenden Kanäle erstrecken, fluidisch verbunden sind; und
sich eins des einen oder der mehreren Abstandselemente (142a-f) an einer Position befindet, die das eine oder die mehreren Verbindungslöcher (112a) überlappt.

14. Ablationssonde nach einem vorhergehenden Anspruch, wobei das Zuleitungskabel (104) einen Abschnitt seiner Länge mit einer relativ größeren Flexibilität verglichen mit einem benachbarten proximalen Abschnitt seiner Länge umfasst, wobei sich der Abschnitt mit der größeren Flexibilität in einem distalen Bereich des Zuleitungskabels (104) befindet, und wobei optional der Abschnitt mit der größeren Flexibilität durch einen geschwächten Abschnitt eines Außenleiters (104b) gebildet ist, der das Zuleitungskabel (104) bildet, und wobei optional der Abschnitt mit der größeren Flexibilität eine Vertiefung oder einen Schlitz umfasst, die bzw. der in dem Außenleiter des Zuleitungskabels (104) gebildet ist, wobei sich die Vertiefung oder der Schlitz entlang eines spiralförmigen Weges entlang der Länge des Zuleitungskabels (104) erstreckt.

15. Ablationssonde nach einem vorhergehenden Anspruch, wobei:
sich der Applikator zwischen einem distalen Ende und einem proximalen Ende erstreckt; und
die Ablationssonde ferner einen Abschnitt mit variierender Flexibilität umfasst, der sich von einer Position neben dem distalen oder proximalen Ende des Applikators (102) erstreckt, wobei der Abschnitt mit variierender Flexibilität einen Bereich der Ablationssonde umfasst, der ein Mindestmaß an Flexibilität nahe dem Applikator (104) hat und der in eine Richtung, die sich entlang der Länge der Ablationssonde von dem Applikator (104) weg erstreckt, an Flexibilität zunimmt, und
wobei optional der Abschnitt mit variierender Flexibilität von einem Bereich der Ablationssonde gebildet wird, der ein Verstärkungsmaterial umfasst, das Verstärkungsmaterial dazu angeordnet ist, in Form und/oder Verteilung zu variieren, um die Flexibilität des Abschnitts mit variierender Flexibilität zu variieren, und wobei optional das Verstärkungsmaterial eine spiralförmige Faser oder einen Draht mit variierender Steigung umfasst, wobei die Steigung entlang der Länge der Ablationssonde in eine Richtung in Richtung des Applikators abnimmt.

## Revendications

1. Sonde d'ablation (100, 200), comprenant :
un applicateur (102) agencé pour appliquer un rayonnement afin de chauffer un tissu environnant ;
un câble d'alimentation (104) agencé pour fournir de l'énergie électromagnétique à l'applicateur (102) ;
un chemin d'écoulement de réfrigérant (106, 108) formant un circuit d'alimentation en réfrigérant ;
un élément tubulaire (112) abritant au moins une partie du câble d'alimentation (104) ;
et un corps d'accouplement (114), le corps d'accouplement (114) comprenant :
une cavité (116) dans laquelle est au moins partiellement encapsulé l'applicateur (102) ;
une interface d'accouplement (118) au niveau de laquelle le corps d'accouplement (114) est accouplé à l'élément tubulaire (112) ;
et un embout distal pointu (114a) adapté pour percer les tissus, le corps d'accouplement (114) étant conçu pour accoupler structurellement l'applicateur (102) et l'élément tubulaire (112) ensemble, dans laquelle le corps d'accouplement (114) est formé de nylon ou de nylon renforcé de verre, et dans laquelle :
i) l'élément tubulaire (112) est formé au moins en partie d'un matériau polymère ; et/ou
ii) la sonde d'ablation comprend en outre un élément déformable (110, 210) entourant au moins une partie de l'élément tubulaire (112), l'élément déformable (110, 210) étant agencé pour se déplacer entre une configuration d'insertion et une configuration déployée, et dans laquelle une partie du chemin de réfrigérant (106, 108) est délimitée entre l'élément déformable (110, 210) et l'élément tubulaire (112) lorsque l'élément déformable est dans la configuration déployée, et dans laquelle le corps d'accouplement (114) comprend une interface d'accouplement (120) au niveau de laquelle le corps d'accouplement (114) est accouplé à l'élément déformable (110), l'élément déformable (110, 210) étant formé au moins en partie de nylon.

2. Sonde d'ablation selon la revendication 1, l'un quelconque du ou des éléments parmi :
b) le corps d'accouplement (114) est formé au moins en partie d'un matériau ayant une résistance au choc sur éprouvette entaillée Charpy dans la plage entre 1 et 50 kJ/m² ;
c) le corps d'accouplement (114) est formé au moins en partie d'un matériau ayant une dureté Rockwell (échelle m) dans la plage entre 10 et 50 ;
d) le corps d'accouplement (114) est formé au moins en partie d'un matériau ayant un module de flexion dans la plage entre 1 et 50 GPa ;
e) le corps d'accouplement (114) est formé au moins en partie d'un matériau ayant une température de déflexion thermique dans la plage entre 80 et 400 degrés Celsius à 1,8 MPa ;
f) le corps d'accouplement (114) est formé au moins en partie d'un matériau transparent ;
g) lorsque la sonde d'ablation comprend l'élément déformable (110, 210), l'élément déformable (110, 210) et au moins une partie du corps d'accouplement (114) formant l'interface d'accouplement (120) au niveau de laquelle le corps d'accouplement (114) est accouplé à l'élément déformable (110, 210) sont tous deux formés à partir de matériaux compatibles pour un thermosoudage ; et
h) dans laquelle le corps d'accouplement (114) est formé d'un matériau ayant une constante diélectrique supérieure à 5, et de préférence supérieure à 20.

3. Sonde d'ablation selon la revendication 1 ou la revendication 2, dans laquelle l'applicateur (102) comprend un corps d'applicateur (102a) et un conducteur d'antenne (124), dans laquelle le corps d'applicateur (102a) comprend une surface externe présentant un canal (126) dans lequel est reçu le conducteur d'antenne (124).

4. Sonde d'ablation selon la revendication 3, dans laquelle le canal (126) s'étend le long d'un chemin hélicoïdal autour d'un axe central du corps d'applicateur (102a).

5. Sonde d'ablation selon la revendication 1 ou la revendication 2, dans laquelle l'applicateur (102) comprend un corps d'applicateur (102a) et un conducteur d'antenne (124), dans laquelle le conducteur d'antenne (124) est agencé sur une surface externe du corps d'applicateur (102a) et dans laquelle un matériau d'empotage ou un adhésif (115) est prévu pour assujettir le conducteur d'antenne (124) en position, et éventuellement dans laquelle le conducteur d'antenne (124) s'étend le long d'un chemin hélicoïdal autour de la surface externe de l'applicateur (102).

6. Sonde d'ablation selon la revendication 3 ou la revendication 4 ou la revendication 5, dans laquelle le câble d'alimentation (104) comprend un conducteur interne (104a), un conducteur externe (104b) et un matériau diélectrique entre eux, et dans laquelle le conducteur d'antenne (124) est formé à partir d'une partie du conducteur interne (104a) du câble d'alimentation (104).

7. Sonde d'ablation selon la revendication 6, dans laquelle une partie de la longueur du conducteur d'antenne (124) s'étend dans une direction distale à partir d'une extrémité distale du conducteur externe (104b) et à l'intérieur d'un trou traversant axial (130) formé dans le corps d'antenne (102a).

8. Sonde d'ablation selon l'une quelconque revendication précédente, présentant l'un quelconque du ou des éléments parmi :
a) le corps d'accouplement (114) est moulé par insertion autour de l'applicateur (104) ;
b) l'interface d'accouplement (118) au niveau de laquelle le corps d'accouplement (114) est accouplé à l'élément tubulaire (112) comprend une partie de chevauchement (118a) du corps d'accouplement (114) qui s'étend à l'intérieur ou autour de l'élément tubulaire (112) de sorte qu'ils se chevauchent l'un l'autre ;
c) un joint soudé ou refondu est formé entre le corps d'accouplement (114) et l'élément tubulaire (112) ;
d) une partie du chemin d'écoulement de réfrigérant (106, 108) est délimitée par un espace entre le câble d'alimentation (104) et l'élément tubulaire (112) ;
e) le câble d'alimentation (104) comprend une partie de réduction de contrainte (408) s'étendant sur la longueur du câble d'alimentation (104) à partir d'une position située au niveau du point de connexion entre le câble d'alimentation (104) et l'applicateur (102) ou à proximité de celui-ci, dans laquelle la partie de réduction de contrainte (408) est formée par une longueur du câble d'alimentation (104) dont la flexibilité diminue lors d'un déplacement sur la longueur du câble d'alimentation (104) dans la direction distale vers l'applicateur (102) ; et/ou
f) un, ou le, composé d'empotage ou adhésif (115) est placé entre l'applicateur (102) et le corps d'accouplement (114).

9. Sonde d'ablation selon une quelconque revendication précédente, comprenant en outre un élément de gaine (250) mobile entre une première position dans laquelle il entoure l'embout pointu (114a, 214a) du corps d'accouplement (114) et une seconde position dans laquelle l'embout pointu (114a, 214a) est découvert.

10. Sonde d'ablation selon la revendication 9, dans laquelle l'élément de gaine (250) s'étend en partie sur la longueur de la sonde d'ablation entre les extrémités distale et proximale de la sonde d'ablation, et dans laquelle la sonde d'ablation comprend une ou plusieurs lignes de commande (252) connectées à l'élément de gaine (250), les lignes de commande (252) s'étendant sur la longueur de la sonde d'ablation entre l'élément de gaine (250) et une position à l'extrémité proximale de la sonde d'ablation ou à proximité de celle-ci,
et éventuellement, la ou les lignes de commande (252) sont connectées à une extrémité distale de l'élément de gaine (250) ou à proximité de celle-ci,
et en outre, éventuellement, l'élément de gaine (250) est formé d'un tube ayant un anneau de renforcement (254) à son extrémité distale, la ou les lignes de commande (252) étant connectées à l'anneau de renforcement (254).

11. Sonde d'ablation selon la revendication 10, dans laquelle la sonde d'ablation est accouplée de manière coulissante à une poignée (201) prévue à son extrémité proximale ou à proximité de celle-ci, et dans laquelle une ou plusieurs lignes de commande (252) sont connectées entre la poignée (201) et l'élément de gaine (250), et les lignes de commande (252) sont agencées pour restreindre la plage de mouvement de l'élément de gaine (250) dans une direction distale à l'écart de la poignée (201).

12. Sonde d'ablation selon l'une quelconque des revendications 9 à 11, l'un quelconque du ou des éléments parmi :
a) lorsqu'elle dépend de la revendication 10, la sonde d'ablation comprend un tube de cathéter externe dans lequel une partie du chemin d'écoulement de réfrigérant est contenue, et dans laquelle les une ou plusieurs lignes de commande (252) s'étendent à l'intérieur d'un canal ou d'une lumière formés dans le tube de cathéter externe ;
b) l'élément de gaine (250) forme un ajustement par friction avec le corps de la sonde d'ablation autour duquel il s'étend et/ou comprend un élément de sollicitation (258) agencé pour solliciter l'élément de gaine (250) dans une direction distale ; et/ou
c) l'élément de gaine (250) comprend un élément de recouvrement (260) agencé pour recouvrir l'embout pointu (114a, 214a) lorsque l'élément de gaine (250) est dans la première position, l'élément de recouvrement (260) étant percé par l'embout pointu (114a, 214a) lorsque l'élément de gaine (250) est déplacé de la première position à la seconde position pour exposer l'embout pointu (114a, 214a).

13. Sonde d'ablation selon une quelconque revendication précédente, comprenant en outre un ou plusieurs éléments d'espacement (142a à f) agencés chacun pour espacer les parois intérieures d'un canal ou de canaux formant le chemin d'écoulement de réfrigérant (106, 108) ; et éventuellement dans laquelle un ou les deux éléments parmi :
a) le canal ou les canaux formant le chemin d'écoulement de réfrigérant (106, 108) ont une section transversale généralement annulaire, et au moins une partie de chacun des un ou plusieurs éléments d'espacement (142a à f) a une forme généralement hélicoïdale ; et/ou
b) le chemin d'écoulement de réfrigérant (106, 108) comprend un premier chemin d'écoulement de réfrigérant(106) et un second chemin d'écoulement de réfrigérant (108), les premier et second chemins d'écoulement de réfrigérant étant en liaison fluidique par un ou plusieurs trous de raccordement (112a) s'étendant à travers des parois respectives des canaux formant les premier et second chemins d'écoulement de réfrigérant (106, 108) ; et
un des un ou plusieurs éléments d'espacement (142a à f) est situé au niveau d'une position chevauchant les un ou plusieurs trous de connexion (112a).

14. Sonde d'ablation selon une quelconque revendication précédente, dans laquelle le câble d'alimentation (104) comprend une partie de sa longueur ayant une flexibilité relativement plus grande par comparaison avec une partie proximale adjacente de sa longueur, la partie de flexibilité plus grande étant située dans une région distale du câble d'alimentation (104), et éventuellement dans laquelle la partie de flexibilité plus grande est formée par une partie affaiblie d'un conducteur externe (104b) formant le câble d'alimentation (104), et éventuellement
dans laquelle la partie de flexibilité plus grande comprend une indentation ou une fente formée dans le conducteur externe du câble d'alimentation (104), l'indentation ou fente s'étendant le long d'un chemin hélicoïdal sur la longueur du câble d'alimentation (104).

15. Sonde d'ablation selon une quelconque revendication précédente, dans laquelle :
l'applicateur s'étend entre une extrémité distale et une extrémité proximale ; et
la sonde d'ablation comprend en outre une partie à flexibilité variable s'étendant à partir d'une position adjacente à l'extrémité distale ou proximale de l'applicateur (102), la partie à flexibilité variable comprenant une région de la sonde d'ablation qui présente un degré minimum de flexibilité au plus près de l'applicateur (104) et dont la flexibilité augmente dans une direction s'étendant sur la longueur de la sonde d'ablation en s'éloignant de l'applicateur (104), et éventuellement
dans laquelle la partie à flexibilité variable est formée par une région de la sonde d'ablation comprenant un matériau de renforcement, le matériau de renforcement étant agencé pour varier en forme et/ou en distribution afin de faire varier la flexibilité de la partie à flexibilité variable, et éventuellement dans laquelle le matériau de renforcement comprend une fibre hélicoïdale ou un fil hélicoïdal ayant un pas variable, le pas diminuant sur la longueur de la sonde d'ablation dans une direction vers l'applicateur.
